(19) Europäisches Patentamt
European Patent Office
Office européen des brevets

(11) Numéro de publication : **0 175 399**
**B1**

(12) # FASCICULE DE BREVET EUROPÉEN

(45) Date de publication du fascicule du brevet : **30.11.88**

(51) Int. Cl.⁴ : **C 07 C 1/24**, C 07 C 11/04

(21) Numéro de dépôt : **85201301.0**

(22) Date de dépôt : **13.08.85**

(54) **Procédé d'obtention d'éthylène à partir d'éthanol.**

(30) Priorité : **28.08.84 LU 85515**

(43) Date de publication de la demande :
**26.03.86 Bulletin 86/13**

(45) Mention de la délivrance du brevet :
**30.11.88 Bulletin 88/48**

(84) Etats contractants désignés :
**AT BE CH DE FR GB IT LI NL SE**

(56) Documents cités :
**EP-A- 0 022 640**
**EP-A- 0 104 800**
**FR-A- 2 240 281**
**FR-A- 2 306 961**
**US-A- 4 011 278**
**Le dossier contient des informations techniques présentées postérieurement au dépôt de la demande et ne figurant pas dans le présent fascicule.**

(73) Titulaire : **De Belgische Staat L'Etat Belge représenté par le Secrétaire général des Services de Programmation**
**de la politique scientifique Rue de la Science, 8**
**B-1040 Bruxelles (BE)**

(72) Inventeur : **Jacobs, Julia M.**
**St. Jozeflei, 24**
**B-2140 Malle (BE)**
Inventeur : **Jacobs, Pierre A.**
**Strijlandstraat, 104**
**B-1686 Gooik (BE)**
Inventeur : **Uytterhoeven, Jan B.**
**Rotspoelstraat, 3**
**B-3300 Heverlee (BE)**

(74) Mandataire : **De Palmenaer, Roger et al**
**Bureau Vander Haeghen 63, avenue de la Toison d'Or**
**B-1060 Bruxelles (BE)**

**EP 0 175 399 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention est relative à un procédé d'obtention d'éthylène à partir d'éthanol anhydre ou aqueux à l'aide d'un catalyseur du type zéolite contenant un silicate d'un métal M1 ayant une valence égale à 3 et une coordination tétraédrique et contenant éventuellement un autre métal M2 compensateur de charge choisi parmi les éléments des groupes Ia, Ib, IIA, IIb, IIIa, IIIb, IVa, IVb, Va, VIb, VIIb et VIII du tableau de Mendeljev. Comme exemples de métaux M2, on peut citer les métaux alcalins, les métaux alcalino-terreux et les lanthanides.

Il est connu, notamment par le brevet U.S. n° 3 894 107, d'utiliser des catalyseurs du type aluminosilicate zéolitique cristallins présentant de faibles teneurs en alumine, notamment des rapports silice/alumine d'au moins 12 pour la transformation de divers composés organiques, tels que de l'éthanol, en hydrocarbures aliphatiques, tels que de l'éthylène.

On connaît, par ailleurs, par la demande de brevet européen n° 0 022 640, un procédé pour transformer de l'éthanol et de l'éthylène en hydrocarbures aliphatiques supérieurs et/ou pour transformer de l'éthanol en éthylène, dans lequel on met l'éthanol en contact avec un catalyseur zéolitique ayant un rapport silice/alumine supérieur à 10, ce catalyseur étant un catalyseur ZSM-5 que l'on traite par un halogénure d'hydrogène, un halogénure organique capable de libérer un halogénure d'hydrogène ou un acide minéral, dans le but d'améliorer la performance catalytique du zéolite.

On connaît également, par le brevet français n° 2 306 961 ou les brevets U.S. correspondants n° 4 025 575 et 4 025 576, un procédé de transformation en mélanges d'oléfines d'une charge d'un alcool et/ou d'un éther ne contenant pas plus de 4 atomes de carbone par groupe alkyle, par contact de cette charge qui peut être une charge d'éthanol avec un catalyseur comprenant un aluminosilicate zéolitique cristallin présentant un indice de contrainte compris entre 1,0 et 12,0, un rapport silice/alumine compris entre 12 et 3 000 et une densité cristalline, sous forme protonée, peu inférieure à 1,6, le contact entre la charge et le catalyseur étant effectué dans des conditions de sévérité suffisamment faibles pour réduire la capacité du catalyseur à produire une aromatisation. Les produits obtenus par ce procédé contiennent essentiellement des mélanges d'oléfines en $C_2$ à $C_5$ quel que soit l'alcool ou l'éther de départ.

Dans ces procédés connus, on obtient soit des hydrocarbures aliphatiques contenant un plus grand nombre d'atomes de carbone que l'alcool de départ, soit un mélange d'hydrocarbures contenant notamment plusieurs oléfines en proportions notables.

On a découvert à présent que certains catalyseurs du type zéolitique cristallins permettent d'obtenir de l'éthylène pratiquement pur comme seul hydrocarbure à partir d'éthanol pur ou de solutions aqueuses même très diluées d'éthanol et ceci avec un taux de tranformation de l'éthanol voisin de 100 %.

Par ailleurs, on a constaté que les catalyseurs du type zéolite choisis pour être utilisés dans le procédé suivant l'invention possèdent une stabilité remarquable, même en présence de quantités importantes d'eau dans la liqueur éthanolique de départ.

Le procédé suivant l'invention pour l'obtention d'éthylène à partir d'éthanol anhydre ou aqueux à l'aide d'un catalyseur du type zéolite contenant un silicate d'un métal M1 ayant une valence égale à 3 et une coordination tétraédrique et contenant éventuellement un autre métal M2 compensateur de charge choisi parmi les métaux alcalins, les métaux alcalino-terreux et les lanthanides est essentiellement caractérisé en ce qu'on met de l'éthanol anhydre ou aqueux en contact avec un catalyseur du type précité contenant une proportion de métal M1 telle que le rapport molaire

$$\frac{M1 - M2/n}{Si + M1}$$

(où n est la valence dudit autre métal) en pourcents est au maximum d'environ 1,5 à une température comprise entre 400 et 800 K (127 et 527 °C) et à un débit de 0,05 à 10 heures$^{-1}$, de telle sorte que le taux de transformation de l'éthanol soit voisin de 100 % et que la sélectivité en carbone éthylénique soit au moins égale à environ 99 % du poids.

Selon une particularité de l'invention, on utilise un catalyseur tel que défini ci-dessus dans lequel le métal M1 est de l'aluminium, du bore, du béryllium, du chrome ou du fer ou un mélange d'aluminium et d'au moins un autre de ces métaux.

Le catalyseur employé dans la préférence suivant l'invention contient avantageusement de l'aluminium comme métal M1 et est, de préférence, exempt de métal M2.

En général, le rapport molaire

$$\frac{M1 - M2/n}{Si + M1}$$

du catalyseur utilisé dans le procédé suivant la présente invention est avantageusement compris entre 0,01 % et 1 %.

Les zéolites ont avantageusement une granulométrie d'au moins 0,5 micron, cette granulométrie pouvant varier fortement et atteindre, par exemple, environ 300 microns.

Dans une forme de réalisation préférée, les catalyseurs utilisés conformément à l'invention sont du type ZSM-5, de préférence au moins partiellement sous forme hydrogène.

Bien que les catalyseurs suivant l'invention soient avantageusement du type ZSM-5, comme décrit dans le brevet U.S. n° 3 702 886, ces catalyseurs peuvent également être des types ZSM-11 (comme décrit dans le brevet U.S. n° 3 709 979) et ZSM-48 (comme décrit dans le brevet européen n° 0 015 132), voire même du type « ferrierite » (comme décrit dans le brevet européen n° 0 012 473) et du type « Faujasite » (comme décrit dans le brevet U.S. n° 3 130 007).

La teneur en métal M1 des catalyseurs, exprimée par le rapport molaire précité, représente la quantité de métal M1 présente dans la structure anionique rigide du cristal zéolitique, à l'exception du métal M1 contenu dans le liant ou sous forme cationique ou autre. Cette teneur en métal M1 et/ou M2 est déterminée par analyse, par exemple par absorption atomique.

Lesdits catalyseurs se caractérisent par un caractère hydrophobe qui est avantageux pour la transformation d'éthanol en éthylène.

Le procédé suivant la présente invention permet d'obtenir de l'éthylène sensiblement pur à partir d'éthanol anhydre ou d'éthanol aqueux, par exemple à partir d'alcool industriel titrant environ 96 % en poids d'éthanol ou d'un mélange de fermentation ne pouvant contenir qu'environ 4 % en poids d'éthanol seulement.

Le catalyseur peut être utilisé de diverses manières dans le procédé suivant l'invention. Ainsi, le catalyseur peut se présenter sous forme d'une couche fixe, d'un lit fluidisé ou de diverses couches de catalyseur transporté. Le catalyseur peut être utilisé avec ou sans liant et, le cas échéant, sous forme de produit extrudé.

Quant à l'éthanol anhydre ou aqueux, il est mis en contact avec le catalyseur, en phase gazeuse ou vapeur, éventuellement en présence d'un gaz inerte, tel que l'azote ou l'hélium, ce gaz pouvant être recyclé, si on le désire.

Les termes « taux de transformation », tels qu'ils sont utilisés dans le présent mémoire, désignent le rapport en pourcents de la quantité d'éthanol transformé à la quantité d'éthanol de départ.

Pour obtenir le taux de transformation de l'éthanol voisin de 100 % et la sélectivité en carbone éthylénique au moins égale à environ 99 % en poids conformément à l'invention, il faut régler la température du catalyseur et le débit de la liqueur de départ en fonction du type particulier de catalyseur utilisé.

La température à laquelle est maintenu le catalyseur pour la transformation d'éthanol en éthylène peut varier entre des limites étendues, notamment entre 400 K (127 °C) et 800 K (527 °C), de préférence entre 500 K (227 °C) et 700 K (427 °C), cette température étant la température maximale atteinte dans la couche du catalyseur.

De même, le débit auquel on fait passer la liqueur éthanolique au contact du catalyseur peut également varier entre des limites étendues. Ainsi, ce débit, déterminé par la vitesse spatiale horaire de liquide (VSHL) qui correspond au volume d'éthanol amené en contact avec le catalyseur par heure et par volume de ce catalyseur, peut varier entre 0,05 et 10 heures$^{-1}$, de préférence entre 0,1 et 5,0 heures.

Les valeurs optimales de la température du catalyseur et du débit de la liqueur éthanolique de départ peuvent être aisément déterminées par voie expérimentale, compte tenu de la teneur en éthanol de la liqueur de départ et du type particulier de catalyseur utilisé avec cette liqueur, en vue d'obtenir un taux de transformation voisin de 100 % et une sélectivité en carbone éthylénique d'au moins 99 % en poids qui caractérisent le procédé suivant l'invention.

Lors de l'application industrielle du procédé suivant l'invention, on procède à des essais en augmentant progressivement la température du catalyseur et/ou le débit de la liqueur éthanolique de départ jusqu'à ce que ces valeurs de température et de débit soient telles que tout l'éthanol contenu dans la liqueur éthanolique de départ soit quantitativement déshydraté et que la sélectivité en carbone éthylénique soit au moins égale à environ 99 % en poids.

En aval de la couche de catalyseur, on obtient un mélange d'eau et de phase organique, cette dernière pouvant être aisément séparée de la phase aqueuse par simple refroidissement, de sorte que l'on récupère une phase organique composée presque exclusivement d'éthylène.

La liqueur éthanolique utilisée dans le procédé suivant l'invention peut provenir de la fermentation d'une biomasse ou d'un produit d'un gaz de synthèse dérivé de charbon ou de pétrole contenant de l'éthanol sous forme anhydre ou sous forme plus ou moins concentrée avant de soumettre la liqueur éthanolique au procédé catalytique de transformation d'éthanol en éthylène suivant la présente invention. Ce dernier procédé permet ainsi de substantielles économies d'énergie, puisque la liqueur éthanolique de départ peut présenter une faible teneur en éthanol qui est presque intégralement transformé en éthylène grâce à la sélectivité remarquable des catalyseurs suivant la présente invention.

Dans une forme de réalisation particulièrement avantageuse du procédé suivant l'invention, celui-ci est mis en œuvre sur un milieu de fermentation contenant de faibles quantités d'éthanol, ces quantités pouvant être d'environ de 4 % seulement en poids.

A la connaissance de la demanderesse, on ne connaît pas de catalyseurs capables d'être utilisés avec des milieux de fermentation industriels, de manière à y déshydrater sélectivement et quantitativement l'éthanol en transformant ce dernier avec des rendements remarquables en éthylène.

Le procédé suivant la présente invention peut être mis en œuvre à des pressions de la liqueur

éthanolique du départ et de l'éventuel gaz inerte servant de diluant comprises entre 1 et 10 atmosphères, mais on préfère opérer à la pression atmosphérique.

La teneur en aluminium ou un autre métal M1 des catalyseurs suivant la présente invention a été choisie, à la suite de nombreux essais, de manière à procurer à ces catalyseurs une acidité telle qu'ils catalysent sélectivement la réaction de déshydratation d'éthanol en éthylène, sans que cette acidité soit cependant trop élevée, par exemple, pour catalyser des réactions de cyclisation ou crackage exigeant des énergies d'activation plus importantes. Les catalyseurs utilisés dans le procédé suivant l'invention ne subissent pas de dégénération par dépôt de coke et se caractérisent par une stabilité thermique et hydrothermique élevée. Même en présence de quantités d'eau, les catalyseurs ne subissent pas une perte d'activité catalytique ou de sélectivité pendant une durée d'utilisation prolongée.

Les exemples suivants illustrent l'invention.

Dans ces exemples, il est montré que l'on peut obtenir un taux de transformation de l'éthanol voisin de 100 % et une sélectivité en carbone éthylénique au moins égale à environ 99 % en poids, chaque fois que l'on utilise un catalyseur du type zéolite cristallin dans lequel le rapport

$$\frac{M1 - M2/n}{Si + M1}$$

est inférieur ou égal à 1,5 %. Par contre, de nombreux exemples révèlent qu'il n'est pas possible d'obtenir un tel taux de transformation quantitatif en même temps qu'une telle sélectivité de plus de 99 % en poids, lorsqu'on fait usage d'un catalyseur dans lequel le rapport susdit est supérieur à 1,5 %.

L'homme de métier ne pouvait pas prévoir qu'en choisissant un catalyseur du type zéolite tel que défini plus haut, il serait possible d'obtenir un taux de transformation de l'éthanol proche de 100 % et une sélectivité en carbone éthylénique de plus de 99 % en poids, dans tous les cas où, dans le catalyseur utilisé, le rapport susdit est égal ou inférieur à 1,5 %. Ce taux de transformation et cette sélectivité remarquables peuvent être obtenus même lorsque ledit rapport est largement inférieur à 1,5 %, par exemple de 0,1 % ou même moins, quelle que soit la teneur en éthanol de la liqueur éthanolique utilisée.

Exemple 1 (comparatif) : Préparation d'un catalyseur H-ZSM-5 présentant un rapport molaire (M1 — M2/n)/(Si + M1) de 1,62 %

Une solution aqueuse de silicate de sodium (source de silice) a été ajoutée à une solution aqueuse d'hydroxyde de tétrapropylammonium (TPOH) pour former une solution 1 formée de 40 ml de solution de silicate de sodium et 1,89 g d'hydroxyde de tétrapropylammonium dans 38 ml d'eau.

Cette solution 1 a été ajoutée, en agitant vigoureusement, à une solution 2 formée de 0,333 g d'aluminate de sodium dissous dans 10 ml d'eau.

Le pH du mélange a été réglé à une valeur de 10 à l'aide d'acide sulfurique concentré, après quoi on a ajouté 40 ml d'eau.

On a ainsi obtenu un gel ayant la composition molaire suivante :

0,5 ($R_2O$) ; 5 ($Na_2O$) ; 0,2 ($Al_2O_3$) ; 24 ($SiO_2$) ; 1 000 ($H_2O$)

dans laquelle R représente le cation organique tétrapropylammonium (TPA).

Ce gel a été agité dans des autoclaves à une température de 432 K (159 °C) pendant 3 jours, après quoi le produit a été lavé, séché à l'air et calciné à l'air à une température de 823 K (550 °C) pendant 12 heures.

Le zéolite obtenu a été ensuite soumis à un échange ionique à 353 K (80 °C) à l'aide d'acide chlorhydrique 0,5 M en utilisant un rapport liquide/solide de 50 et en agitant de manière continue pendant 1 heure.

Après lavage et séchage, on a obtenu un catalyseur cristallin hydrophobe ayant un rapport Al/Si + Al de 1,62 % et se présentant sous forme de cristaux d'une taille d'environ 4 microns. Comme ce catalyseur ne contient pas de sodium (M2), ce rapport est égal à (M1)/(Si + M1), ou M1 = Al.

Exemple 2 (comparatif) : Transformation d'éthanol à l'aide du catalyseur de l'exemple 1

Un échantillon du catalyseur de l'exemple 1 a été utilisé pour la réaction de déshydratation d'éthanol en éthylène en présence d'eau.

Le catalyseur a été chargé dans un réacteur tubulaire à flux continu et utilisé sans prétraitement.

Comme solution de départ, on a utilisé une solution aqueuse contenant 90 % en poids d'éthanol absolu titrant plus de 99,5 % d'éthanol (éthanol U.C.B., Belgique).

On a fait passer cette solution en phase gazeuse sur le catalyseur à une vitesse spatiale horaire liquide (VSHL) de 0,31 $heure^{-1}$ conjointement avec 15 moles d'hélium sec comme diluant par mole d'éthanol. La durée de contact du réactif avec le catalyseur a été de 1,8 seconde.

La réaction a été exécutée à la pression atmosphérique et l'on a fait varier la température par paliers distincts depuis environ 450 K (177 °C) jusqu'à 570 K (237 °C).

Les produits de réaction ont été analysés par chromatographie gazeuse en continu.

On a constaté que le catalyseur n'a subi aucune désactivation pendant le traitement.

Les conditions opératoires et les résultats des analyses sont indiqués dans le tableau 1 suivant. Ce tableau indique notamment :
— les températures ;
— le débit du réactif VSHL ;
— le pourcentage de transformation ;
— les répartitions en % en poids des hydrocarbures.
Le pourcentage de transformation a été exprimé comme suit :

$$\frac{\text{quantité en grammes de } C_2H_5OH \text{ transformé}}{\text{quantité en grammes de } C_2H_5OH \text{ fourni}} \times 100$$

Tableau 1

Catalyseur : H-ZSM-5 (Al/Si + Al % = 1,62)

| | | | | | | |
|---|---|---|---|---|---|---|
| **Conditions de réaction** | | | | | | |
| Température (K) | 449 | 472 | 496 | 520 | 544 | 569 |
| °C | 176 | 199 | 223 | 247 | 271 | 296 |
| VSHL (1/hr) | 0,31 | 0,31 | 0,31 | 0,31 | 0,31 | 0,31 |
| Transformation (%) | 32,2 | 50,3 | 70,0 | 99,6 | 99,7 | 99,8 |
| **Distribution des produits (% en poids)** | | | | | | |
| Ether diéthylique | 75,9 | 57,9 | 22,2 | 0,0 | 0,0 | 0,0 |
| Eau | 20,7 | 25,1 | 33,7 | 39,1 | 39,2 | 39,2 |
| Hydrocarbures | 3,4 | 17,0 | 44,1 | 60,9 | 60,8 | 60,8 |
| CO* | 0,0 | 0,0 | 0,0 | 0,0 | 0,0 | 0,0 |
| $CO_2$* | 0,0 | 0,0 | 0,0 | 0,0 | 0,0 | 0,0 |
| **Distribution des hydrocarbures (% en poids)** | | | | | | |
| Méthane | 0,0 | 0,0 | 0,0 | 0,0 | 0,0 | 0,0 |
| Ethylène | 90,7 | 97,4 | 98,0 | 73,2 | 55,6 | 43,5 |
| Ethane | 0,0 | 0,0 | 0,0 | 0,0 | 0,0 | 0,0 |
| Propylène + propane | 0,0 | 0,0 | 0,2 | 3,7 | 6,2 | 8,7 |
| Butanes | 2,2 | 0,6 | 0,6 | 2,4 | 3,8 | 5,9 |
| Butènes | 0,0 | 0,1 | 0,2 | 5,0 | 8,7 | 10,7 |
| Pentanes | 0,0 | 0,0 | 0,1 | 1,9 | 2,7 | 3,5 |
| Pentènes | 0,0 | 0,0 | 0,0 | 3,2 | 5,5 | 6,0 |
| C5+ | 7,1 | 1,9 | 0,9 | 10,6 | 17,5 | 21,7 |
| % iso en C4 | 100,0 | 82,1 | 55,5 | 57,9 | 61,4 | 64,8 |
| % aromatiques en C5+ | 89,0 | 84,1 | 76,1 | 24,9 | 39,7 | 49,0 |

* Composés non détectés

La transformation théorique maximale d'éthanol en hydrocarbures et eau est respectivement de 61 % en poids et de 39 % en poids. Le tableau 1 montre que ce résultat est obtenu à une température de 520 K (247 °C).

A des températures plus élevées, le taux de transformation d'éthanol en hydrocarbures s'est maintenu, mais des quantités plus élevées de produits autres que l'éthylène se sont formées.

La sélectivité du catalyseur pour la formation d'éthylène n'était que relativement élevée avec un maximum de 98 % en poids et un taux de transformation de 70 % à une température de 496 K (223 °C).

Aux taux de transformations plus élevés, la formation de sous-produits (autres que l'éthylène) est devenue importante et la sélectivité du catalyseur vis-à-vis de la formation d'éthylène a diminué rapidement.

Le catalyseur utilisé dans cet exemple, dans lequel le rapport Al/Si + Al n'est cependant que peu supérieur au maximum de 1,5 %, ne permet pas d'obtenir à la fois un taux de transformation d'éthanol voisin de 100 % et une sélectivité en carbone éthylénique supérieure à 99 % en poids.

Exemple 3 : Préparation d'un catalyseur H-ZSM-5 présentant un rapport (M1 — M2/n)/(Si + M1) de 1,0 %

Les solutions de départ suivantes ont été utilisées :
solution 1 :

(a) 20 grammes de $SiO_2$ (aérosil) dans 830 ml de bromure de tétrapropylammonium 1 N (TPABr) ;
(b) 5,32 grammes de NaOH dans 10 ml d'eau ;
(c) 4,66 grammes de $NH_4OH$ dans 10 ml d'eau.
solution 2 : 1,24 g de $Al(NO_3)_3 \cdot 9H_2O$ dans 10 ml d'eau.
solution 3 : 610 g de glycérol.

A la solution 1, obtenue en mélangeant (a) et (b), puis (c), on a d'abord ajouté la solution 2, puis la solution 3 en agissant de manière continue.

Le mélange obtenu a été cristallisé dans des autoclaves pendant un minimum de 4 à 5 jours à une température de 423 K (150 °C) tout en agitant.

Le produit solide obtenu a été séparé de la solution liquide surnageante par filtration, puis lavé à l'eau et séché.

Le produit a été analysé par diffraction aux rayons X et on a constaté qu'il s'agissait de 100 % de zéolite ZSM-5.

La composition du mélange réactionnel, sur une base molaire, était la suivante :
100 $SiO_2/Al(NO_3)_3$ ; 13 NAOH ; 83 TPABr ; 13 $NH_4OH$ ; 662 $C_3H_5(OH)_3$.

Le produit alcalin a été activé en éliminant le cation organique TPA, par calcination à l'air à 823 K (550 °C) pendant 12 heures.

La forme H du catalyseur a été obtenue par échange ionique avec de l'acide chlorhydrique 0,5 M à une température de 353 K (80 °C) pendant 1 heure, en agitant. Le rapport liquide/solide était de 50.

Le catalyseur sous forme hydrogène obtenu par filtration sous vide, lavage et séchage, se présentait sous forme de cristaux de 5 à 6 microns. Sur base des résultats d'absorption atomique, le catalyseur ne contenait pas de sodium (M2). Le rapport molaire (M1)/(Si + M1), où M1 = Al est de 1,0 %.

Exemple 4 : Transformation d'éthanol en éthylène à l'aide du catalyseur de l'exemple 3

On a fait passer une solution à 90 % en poids d'éthanol et 10 % en poids d'eau sur un échantillon du catalyseur décrit dans l'exemple 3, dans des conditions de réaction similaires à celles décrites dans l'exemple 2.

Les conditions opératoires et les produits formés à six températures différentes sont indiqués dans le tableau 2 suivant :

Tableau 2

Catalyseur : H-ZSM-5 (Al/Si + Al % = 1,0)

| | | | | | | |
|---|---|---|---|---|---|---|
| Conditions de réaction | | | | | | |
| Température (K) | 449 | 474 | 498 | 521 | 544 | 566 |
| °C | 176 | 201 | 225 | 248 | 271 | 293 |
| VSHL (1/hr) | 0,31 | 0,31 | 0,31 | 0,31 | 0,31 | 0,31 |
| Transformation (%) | 16,4 | 32,9 | 60,9 | 100,0 | 100,0 | 100,0 |
| Distribution des produits (% en poids) | | | | | | |
| Ether diéthylique | 80,4 | 71,1 | 21,0 | 0,0 | 0,0 | 0,0 |
| Eau | 19,6 | 21,8 | 34,0 | 39,1 | 39,0 | 39,1 |
| Hydrocarbures | 0,0 | 7,1 | 45,0 | 60,9 | 61,0 | 60,9 |
| CO* | 0,0 | 0,0 | 0,0 | 0,0 | 0,0 | 0,0 |
| $CO_2$* | 0,0 | 0,0 | 0,0 | 0,0 | 0,0 | 0,0 |
| Distribution des hydrocarbures (% en poids) | | | | | | |
| Méthane | 0,0 | 0,0 | 0,0 | 0,0 | 0,0 | 0,0 |
| Ethylène | 0,0 | 99,1 | 99,5 | 99,6 | 88,6 | 77,2 |
| Ethane | 0,0 | 0,0 | 0,0 | 0,0 | 0,0 | 0,0 |
| C3 + C4 | 0,0 | 0,9 | 0,1 | 0,0 | 7,6 | 11,8 |
| Pentanes | 0,0 | 0,0 | 0,0 | 0,1 | 1,0 | 1,1 |
| Pentènes | 0,0 | 0,0 | 0,0 | 0,0 | 1,6 | 2,0 |
| C5+ | 0,0 | 0,0 | 0,4 | 0,3 | 1,2 | 7,9 |
| % aromatiques en C5+ | 0,0 | 0,0 | 0,0 | 0,0 | 0,0 | 83,0 |

* Composés non détectés

En comparant les résultats de l'utilisation du catalyseur H-ZSM-5 ayant un rapport Al/Si + Al de 1,0 % (tableau 2) à ceux de l'utilisation du catalyseur H-ZSM-5 ayant un rapport Al/Si + Al de 1,62 %

(exemple 2-tableau 1), on constate que la sélectivité du catalyseur de l'exemple 3 vis-à-vis de la formation d'éthylène est plus élevée et que la formation de sous-produits est moindre. La sélectivité maximale (99,6 %) pour $C_2H_4$ (éthylène) s'est manifestée à une température de 521 K (248 °C) avec un taux de transformation de 100 %.

Exemple 5 : Préparation d'un catalyseur H-ZSM-5 présentant un rapport 5M1 — M2/n)/(Si + M1) de 0,2 %

Ce catalyseur a été préparé de la manière décrite dans l'exemple 3, en modifiant seulement la teneur en aluminium, par addition de la quantité réglée de $Al(NO_3)_3 \cdot 9H_2O$ aux mêmes quantités de $SiO_2$, NaOH et TPABr.

La composition molaire du mélange de synthèse était la suivante :

500 $SiO_2/Al(NO_3)_3$ ; 13 NAOH ; 83 TPABr ; 13 $NH_4OH$ ; 662 $C_3H_5(OH)_3$.

L'analyse aux rayons X a montré que le produit était du zéolite ZSM-5 à 100 %.

L'échange ionique avec le cation ammonium a été obtenu en chauffant le catalyseur au reflux dans un excès de chlorure d'ammonium 1N, pendant 1 heure.

La forme hydrogène du catalyseur a été obtenue en calcinant le catalyseur à l'air à 723 K (450 °C) pendant 1 heure.

On a ainsi obtenu un catalyseur H-ZSM-5 sous forme de cristaux de 3 à 5 microns, ne contenant pas de sodium (M2) et ayant un rapport molaire (M1)/(Si + M1) égal à 0,2 %, où M1 = Al.

Exemple 6 : Transformation d'éthanol en éthylène à l'aide du catalyseur de l'exemple 5

On a opéré dans les mêmes conditions que dans l'exemple 2, en utilisant comme réactif de départ une solution aqueuse à 90 % en poids d'éthanol.

Le tableau 3 suivant indique les conditions opératoires et les produits obtenus à différentes températures de réaction.

Tableau 3

Catalyseur : H-ZSM-5 (Al/Si + Al % = 0,2)

| | | | | | | |
|---|---|---|---|---|---|---|
| **Conditions de réaction** | | | | | | |
| Température (K) | 440 | 463 | 488 | 519 | 544 | 565 |
| °C | 167 | 190 | 215 | 246 | 271 | 292 |
| VSHL (1/hr) | 0,31 | 0,31 | 0,31 | 0,31 | 0,31 | 0,31 |
| Transformation (%) | 9,3 | 26,1 | 47,5 | 100,0 | 100,0 | 100,0 |
| **Distribution des produits (% en poids)** | | | | | | |
| Ether diéthylique | 80,1 | 69,6 | 31,3 | 0,0 | 0,0 | 0,0 |
| Eau | 18,5 | 21,7 | 31,2 | 39,0 | 39,0 | 39,0 |
| Hydrocarbures | 0,0 | 8,1 | 37,1 | 60,8 | 60,8 | 60,8 |
| CO | 1,4 | 0,6 | 0,4 | 0,2 | 0,2 | 0,2 |
| $CO_2$ | 0,0 | 0,0 | 0,0 | 0,0 | 0,0 | 0,0 |
| **Distribution des hydrocarbures (% en poids)** | | | | | | |
| Méthane | 0,0 | 0,0 | 0,0 | 0,0 | 0,0 | 0,0 |
| Ethylène | 0,0 | 100,0 | 100,0 | 99,4 | 91,9 | 85,8 |
| Ethane | 0,0 | 0,0 | 0,0 | 0,0 | 0,0 | 0,0 |
| Propylène + propane | 0,0 | 0,0 | 0,0 | 0,6 | 2,4 | 4,0 |
| Butanes | 0,0 | 0,0 | 0,0 | 0,0 | 0,9 | 1,1 |
| Butènes | 0,0 | 0,0 | 0,0 | 0,0 | 2,6 | 4,8 |
| Pentanes | 0,0 | 0,0 | 0,0 | 0,0 | 0,5 | 0,6 |
| Pentènes | 0,0 | 0,0 | 0,0 | 0,0 | 0,8 | 1,2 |
| C5 + | 0,0 | 0,0 | 0,0 | 0,0 | 0,9 | 2,5 |
| % iso en C4 | 0,0 | 0,0 | 0,0 | 0,0 | 70,2 | 71,0 |
| % aromatiques en C5 + | 0,0 | 0,0 | 0,0 | 0,0 | 59,3 | 87,2 |

Ce tableau 3, comparé aux tableaux des exemples 2 et 4, révèle une sélectivité remarquable (99,4 % en poids) du catalyseur vis-à-vis de la formation d'éthylène, en même temps qu'un taux de transformation quantitatif de l'éthanol lorsqu'on opère à une température de 519 K (246 °C)

Exemple 7 : Préparation d'un catalyseur H-ZSM-5 ayant un rapport (M1 — M2/n)/(Si + M1) de 0,1 %

On a synthétisé ce catalyseur en utilisant les mêmes conditions opératoires que dans l'exemple 1, si ce n'est que l'on a modifié la teneur en aluminium, en changeant la quantité d'aluminate de sodium dans le mélange réactionnel.

La composition molaire du mélange de synthèse était la suivante :

0,5($R_2O$) ; 5($Na_2O$) ; 0,012($Al_2O_3$) ; 24($SiO_2$) ; 1 000($H_2O$)

où R représente le cation tétrapropylammonium.

La calcination et l'échange ionique ont été réalisés de la manière décrite dans l'exemple 1.

L'analyse par diffraction aux rayons X a révélé la présence d'une phase fortement cristalline de ZSM-5.

Ce catalyseur H-ZSM-5 obtenu ne contenait pas de sodium (M2), et le rapport molaire (M1)/(Si + M1) est égal à 0,1 %, où M1 = Al. Le catalyseur se présentait sous forme de cristaux de 3 à 5 microns.

Exemple 8 : Transformation d'éthanol en éthylène à l'aide du catalyseur de l'exemple 7

En opérant dans les mêmes conditions réactionnelles que dans l'exemple 2, on a fait passer une solution aqueuse à 90 % en poids d'éthanol sur un échantillon du catalyseur de l'exemple 7.

Les conditions opératoires et analyses des produits sont données dans le tableau 4 suivant :

Tableau 4

Catalyseur : H-ZSM-5 (Al/Si + Al % = 0,1)

| | | | | | | |
|---|---|---|---|---|---|---|
| **Conditions de réaction** | | | | | | |
| Température (K) | 439 | 473 | 490 | 503 | 551 | 566 |
| °C | 166 | 200 | 217 | 230 | 278 | 293 |
| VSHL (1/hr) | 0,31 | 0,31 | 0,31 | 0,31 | 0,31 | 0,31 |
| Transformation (%) | 13,0 | 33,3 | 62,0 | 96,1 | 100,0 | 100,0 |
| **Distribution des produits (% en poids)** | | | | | | |
| Ether diéthylique | 79,0 | 59,4 | 13,5 | 0,1 | 0,0 | 0,0 |
| Eau | 19,1 | 24,2 | 35,6 | 38,9 | 39,0 | 39,0 |
| Hydrocarbures | 0,8 | 15,8 | 50,6 | 60,8 | 60,8 | 60,8 |
| CO | 1,1 | 0,6 | 0,3 | 0,2 | 0,2 | 0,2 |
| $CO_2$ | 0,0 | 0,0 | 0,0 | 0,0 | 0,0 | 0,0 |
| **Distribution des hydrocarbures (% en poids)** | | | | | | |
| Méthane | 0,0 | 0,0 | 0,0 | 0,0 | 0,0 | 0,0 |
| Ethylène | 50,8 | 94,0 | 98,0 | 99,6 | 99,5 | 97,2 |
| Ethane | 0,0 | 0,0 | 0,0 | 0,0 | 0,0 | 0,0 |
| Propylène + propane | 0,0 | 0,0 | 0,0 | 0,0 | 0,0 | 0,0 |
| Butanes | 0,0 | 0,0 | 1,4 | 0,0 | 0,0 | 0,0 |
| Butènes | 49,2 | 6,0 | 0,6 | 0,4 | 0,0 | 0,2 |
| Pentanes | 0,0 | 0,0 | 0,0 | 0,0 | 0,0 | 0,0 |
| Pentènes | 0,0 | 0,0 | 0,0 | 0,0 | 0,0 | 0,0 |
| C5 + | 0,0 | 0,0 | 0,0 | 0,0 | 0,5 | 2,6 |
| % iso en C4 | 0,0 | 0,0 | 69,1 | 0,0 | 0,0 | 0,0 |
| % aromatiques en C5 + | 0,0 | 0,0 | 0,0 | 0,0 | 100,0 | 100,0 |

Ce tableau 4 montre une sélectivité remarquable (99,5 % en poids) du catalyseur pour la transformation de l'éthylène en même temps qu'un taux de transformation quantitatif à une température de réaction de 551 K (278 °C).

Exemple 9 : Préparation d'un catalyseur H-ZSM-5 ayant un rapport (M1 — M2/n)/(Si + M1) de 0,1 %

On a préparé ce catalyseur de la manière décrite dans l'exemple 3, si ce n'est que sa teneur en aluminium a été adaptée en ajoutant la quantité ajustée de nitrate d'aluminium ($Al(NO_3)_3 \cdot 9H_2O$) à des quantités constantes de $SiO_2$, NaOH et TPABr.

La composition molaire du mélange de synthèse du zéolite ZSM-5 était la suivante :

1 000 $SiO_2/Al(NO_3)_3$ ; 13 NaOH ; 83 TPABr ; 13 NH OH ; 662 $C_3H_5(OH)_3$.

L'analyse par diffraction aux rayons X a révélé que le produit était constitué à 100 % de ZSM-5.

Le produit solide a été calciné et la forme H du zéolite a été préparée par échange ionique avec du HCl, comme décrit dans l'exemple 1.

Exemple 10 : Transformation d'éthanol en éthylène à l'aide du catalyseur de l'exemple 9

On a fait passer, en phase gazeuse, une solution aqueuse diluée d'éthanol, à savoir une solution aqueuse contenant 8,08 % en poids (10 % en volume) d'éthanol sur un échantillon du catalyseur de l'exemple 9, en opérant de la manière décrite dans l'exemple 2, si ce n'est que le débit de la solution éthanolique et, par conséquent, la durée de contact de cette solution avec le catalyseur ont été modifiés comme suit :

VSHL : 1,8 heure$^{-1}$

temps de contact : 0,16 seconde.

Comme diluant, 2 moles d'hélium sec par mole d'éthanol ont été utilisées.

Les résultats obtenus à diverses températures sont indiqués dans le tableau 5 suivant :

Tableau 5

Catalyseur : H-ZSM-5 (Al/Si + Al % = 0,1)

| | | | | | | |
|---|---|---|---|---|---|---|
| **Conditions de réaction** | | | | | | |
| Température (K) | 497 | 581 | 603 | 654 | 665 | 685 |
| °C | 224 | 308 | 330 | 381 | 392 | 412 |
| VSHL (1/hr) | 1,8 | 1,8 | 1,8 | 1,8 | 1,8 | 1,8 |
| Transformation (%) | 2,9 | 48,3 | 65,2 | 79,7 | 83,8 | 100,0 |
| **Distribution des produits (% en poids)** | | | | | | |
| Ether diéthylique | 79,8 | 25,3 | 13,6 | 2,5 | 0,9 | 0,0 |
| Eau | 19,7 | 33,0 | 35,8 | 38,5 | 38,9 | 39,1 |
| Hydrocarbures | 0,5 | 41,7 | 50,6 | 59,0 | 60,2 | 60,9 |
| CO | 0,0 | 0,0 | 0,0 | 0,0 | 0,0 | 0,0 |
| $CO_2$ | 0,0 | 0,0 | 0,0 | 0,0 | 0,0 | 0,0 |
| **Distribution des hydrocarbures (% en poids)** | | | | | | |
| Méthane | 0,0 | 0,0 | 0,0 | 0,0 | 0,0 | 0,0 |
| Ethylène | 100,0 | 100,0 | 100,0 | 100,0 | 100,0 | 99,6 |
| Ethane | 0,0 | 0,0 | 0,0 | 0,0 | 0,0 | 0,0 |
| Propylène + propane | 0,0 | 0,0 | 0,0 | 0,0 | 0,0 | 0,4 |
| Butanes | 0,0 | 0,0 | 0,0 | 0,0 | 0,0 | 0,0 |
| Butènes | 0,0 | 0,0 | 0,0 | 0,0 | 0,0 | 0,0 |
| Pentanes | 0,0 | 0,0 | 0,0 | 0,0 | 0,0 | 0,0 |
| Pentènes | 0,0 | 0,0 | 0,0 | 0,0 | 0,0 | 0,0 |
| C5+ | 0,0 | 0,0 | 0,0 | 0,0 | 0,0 | 0,0 |
| % iso en C4 | 0,0 | 0,0 | 0,0 | 0,0 | 0,0 | 0,0 |
| % aromatiques en C5+ | 0,0 | 0,0 | 0,0 | 0,0 | 0,0 | 0,0 |

Ce tableau révèle que 8,08 % en poids d'éthanol dans de l'eau ont été quantitativement transformés en éthylène avec une sélectivité du catalyseur en carbone éthylénique de 99,6 % en poids à une température de 685 K (412 °C).

Exemple 11 : Essai de durabilité d'un catalyseur H-ZSM-5 ayant un rapport (M1 — M2/n)/(Si + M1) de 0,1 %

Le catalyseur H-ZSM-5 ayant une proportion molaire optimale d'aluminium (Al/Si + Al = 0,1 %) de l'exemple 7 a été soumis à un essai de durée de vie dans les mêmes conditions opératoires que dans l'exemple 2, si ce n'est que la température de réaction a été maintenue constante à 553 K (280 °C).

Le débit du produit éthanolique de départ était de 0,31 heure$^{-1}$.

Comme produit de départ, on a utilisé une solution aqueuse à 90 % en poids d'éthanol.

On a fait passer cette solution sur le catalyseur en phase gazeuse.

Les résultats obtenus dans cet essai sont schématisés dans le tableau 6 suivant :

Tableau 6

Catalyseur : H-ZSM-5 (0,1 % Al/Si + Al)
Réactif : 90 % en poids de EtOH* dans $H_2O$
g réactif/g catalyseur

| | transformation** (%) | $C_2H_4$*** (% en poids) |
|---|---|---|
| 150 g/g | 100,0 | 99,5 |
| 300 g/g | 100,0 | 99,6 |
| 500 g/g | 100,0 | 99,7 |
| 800 g/g | 100,0 | 99,7 |

* EtOH = alcool éthylique
** Transformation de EtOH (%) =

$$\frac{\text{quantité (g) de EtOH transformé}}{\text{quantité (g) de EtOH fourni}} \times 100$$

*** Sélectivité en carbone éthylénique (% en poids) =

$$\frac{\text{quantité (g) de } C_2H_4 \text{ formé}}{\text{quantité (g) d'hydrocarbures formés}} \times 100$$

Le tableau 6 montre que le catalyseur zéolite H-ZSM-5 contenant 1 % de Al/Si + Al se caractérise par le fait que le taux de transformation d'éthanol en éthylène et la sélectivité du catalyseur en carbone éthylénique ne subissent pas d'altération dans le temps.

Ce tableau 6 montre que le catalyseur H-ZSM-5 à 0,1 % de (M1)/(Si + M1), où M1 = Al, présente une stabilité remarquable pour la production sélective d'éthylène à partir d'éthanol en présence d'eau.

Exemple 12 : Préparation d'un catalyseur du type silicalite ayant un rapport (M1 — M2/n)/(Si + M1) de 0,02 %

Ce catalyseur du type zéolite exempt d'aluminium ajouté a été synthétisé par le procédé décrit dans l'exemple 1 du brevet U.S. nº 4.061.724.

Comme ce catalyseur ne contenait pas de sodium (M2), le rapport molaire (M1)/(Si + M1), où M1 = Al est égal à 0,02 %.

Exemple 13 : Transformation d'éthanol en éthylène à l'aide du catalyseur de l'exemple 12

En opérant comme dans l'exemple 2, on a fait passer une solution aqueuse à 90 % en poids d'éthanol sur le catalyseur de l'exemple 12.

Les résultats de cet essai sont montrés dans le tableau 7 suivant :

Tableau 7

Catalyseur : Silicalite (Al/Si + Al % = 0,02)

| | | | | | | |
|---|---|---|---|---|---|---|
| Conditions de réaction | | | | | | |
| Température (K) | 561 | 628 | 651 | 660 | 675 | 708 |
| °C | 288 | 355 | 378 | 387 | 402 | 435 |
| VSHL (1/hr) | 0,31 | 0,31 | 0,31 | 0,31 | 0,31 | 0,31 |
| Transformation (%) | 1,6 | 10,1 | 35,0 | 94,7 | 99,8 | 100,0 |
| Distribution des produits (% en poids) | | | | | | |
| Ether diéthylique | 52,3 | 15,8 | 3,1 | 0,5 | 0,0 | 0,0 |
| Acétaldéhyde | 11,1 | 13,8 | 6,4 | 2,7 | 0,9 | 4,6 |
| Eau | 13,2 | 24,6 | 32,8 | 35,8 | 38,0 | 33,0 |
| Hydrocarbures | 12,1 | 40,1 | 54,7 | 58,8 | 60,3 | 58,3 |
| CO | 11,3 | 3,5 | 1,2 | 0,8 | 0,5 | 1,1 |
| $CO_2$ | 0,0 | 2,2 | 1,8 | 1,4 | 0,3 | 3,0 |

Tableau 7 (Suite)

| | | | | | | |
|---|---|---|---|---|---|---|
| Distribution des hydrocarbures (% en poids) | | | | | | |
| Méthane | 0,0 | 0,0 | 0,0 | 0,0 | 0,0 | 0,2 |
| Ethylène | 100,0 | 100,0 | 99,2 | 99,3 | 99,8 | 98,3 |
| Ethane | 0,0 | 0,0 | 0,0 | 0,0 | 0,0 | 0,0 |
| Propylène + propane | 0,0 | 0,0 | 0,0 | 0,0 | 0,0 | 1,2 |
| Butanes | 0,0 | 0,0 | 0,0 | 0,0 | 0,0 | 0,0 |
| Butènes | 0,0 | 0,0 | 0,8 | 0,7 | 0,2 | 0,3 |
| Pentanes | 0,0 | 0,0 | 0,0 | 0,0 | 0,0 | 0,0 |
| Pentènes | 0,0 | 0,0 | 0,0 | 0,0 | 0,0 | 0,0 |
| C5+ | 0,0 | 0,0 | 0,0 | 0,0 | 0,0 | 0,0 |
| % iso en C4 | 0,0 | 0,0 | 100,0 | 100,0 | 100,0 | 100,0 |
| % aromatiques en C5+ | 0,0 | 0,0 | 0,0 | 0,0 | 0,0 | 0,0 |

Le tableau 7 montre qu'un taux de transformation de l'éthanol de pratiquement 100 % est obtenu à partir de 675 K (402 °C). A cette température, la sélectivité du catalyseur du type zéolite vis-à-vis de la formation d'éthylène est maximale (99,8 % en poids).

Exemple 14 : Essai de durabilité d'un catalyseur H-ZSM-5 ayant un rapport (M1—M2/n)/(Si + M1) de 0,1 % dans la transformation d'un produit éthanolique composé d'une liqueur de fermentation

Le catalyseur H-ZSM-5, ayant une proportion molaire optimale d'aluminium (Al/Si + Al = 0,1 %) de l'exemple 9, a été soumis à un essai de durée de vie.

Un échantillon du catalyseur a été chargé dans le réacteur tubulaire à flux continu et utilisé sans prétraitement.

Comme solution, on fait passer, en phase gazeuse, un produit éthanolique provenant d'une liqueur de fermentation composition suivante :

8 % en volume d'éthanol avec

0,2 ppm d'acétaldéhyde
5,0 ppm de méthanol
0,3 ppm de n-propanol
0,1 ppm d'isobutanol
0,1 ppm de n-butanol
0,1 ppm d'alcool iso-amylique
0,2 ppm d'acétone

La température de réaction a été maintenue constante à 580 K (307 °C).
La vitesse spatiale horaire liquide (VSHL) en éthanol était de 0,25 $heure^{-1}$.
Comme diluant, on a ajouté 2 moles d'hélium sec par mole d'éthanol.
Les résultats des analyses sont indiqués dans le tableau 8.

Tableau 8

Catalyseur : H-ZSM-5 (0,1 % Al/Si + Al)
Réactif : 8 % en volume de EtOH* + impuretés
g réactif/g catalyseur :

| | transformation** (%) | $C_2H_4$*** (% en poids) |
|---|---|---|
| 0,1 g/g | 98 | 99,4 |
| 10,0 g/g | 98 | 99,5 |
| 50,0 g/g | 98 | 99,5 |
| 100,0 g/g | 98 | 99,5 |

* EtOH = alcool éthylique
* Transformation de EtOH (%) =

$$\frac{\text{quantité (g) de EtOH transformé}}{\text{quantité (g) de EtOH fourni}} \times 100$$

* Sélectivité en carbone éthylénique (% en poids) =

$$\frac{\text{quantité (g) de } C_2H_4 \text{ formé}}{\text{quantité (g) d'hydrocarbures formés}} \times 100$$

Le tableau 8 montre que le catalyseur zéolite H-ZSM-5 contenant 0,1 % de Al/Si + Al présente une stabilité élevée pour la production sélective d'éthylène à partir d'une liqueur de fermentation éthanolique filtrée sur charbon actif.

Le taux de transformation d'éthanol en éthylène et la sélectivité en carbone éthylénique du catalyseur ne subissent pas d'altération dans le temps.

Exemple 15 : Préparation d'un catalyseur H-ZSM-5 ayant un rapport (M1 — M2/n)/(Si + M1) de 0,33 %

On a synthétisé ce catalyseur de la manière décrite dans l'exemple 1, si ce n'est que l'on a ajouté, au lieu de l'aluminate de sodium ($NaAlO_2$), le borate de sodium ($NaBO_2$) en quantité réglée dans le mélange réactionnel.

La composition molaire du mélange de synthèse était la suivante :

0,5($R_2O$) ; 5($Na_2O$) ; 0,12($B_2O_3$) ; 24($SiO_2$) ; 1 000($H_2O$)

R représente le cation tétrapropylammonium.

Le produit solide obtenu a été calciné à l'air à 873 K (600 °C) pendant 12 heures.

L'échange ionique a été effectué en chauffant le catalyseur au reflux dans un excès de chlorure d'ammonium de la manière décrite dans l'exemple 5.

Avant l'utilisation comme catalyseur, le zéolite a été activé dans le réacteur même par calcination à 673 K (400 °C) sous un courant d'hélium pendant 1 heure.

L'analyse par R.M.N. du zéolite après la synthèse et le prétraitement a montré que la teneur en bore présente dans la structure anionique zéolitique est de 0,33 % (M1)/(Si + M1) où M1 = B, le catalyseur ne contenant pas de sodium (M2).

Exemple 16 : Transformation d'éthanol en éthylène à l'aide du catalyseur de l'exemple 15

Après le prétraitement précité, on a fait passer une solution aqueuse à 90 % en poids d'éthanol sur un échantillon du catalyseur de l'exemple 15.

Les conditions réactionnelles étaient les mêmes que dans l'exemple 2.

Les résultats obtenus à diverses températures sont indiqués dans le tableau 9 suivant :

Tableau 9

Catalyseur : H-ZSM-5 (B/Si + B % = 0,33)

| | | | | | | |
|---|---|---|---|---|---|---|
| Conditions de réaction | | | | | | |
| Température (K) | 436 | 481 | 507 | 530 | 553 | 600 |
| °C | 163 | 208 | 234 | 257 | 280 | 327 |
| VSHL (1/hr) | 0,31 | 0,31 | 0,31 | 0,31 | 0,31 | 0,31 |
| Transformation (%) | 3,8 | 15,9 | 26,5 | 43,8 | 97,6 | 100,0 |
| Distribution des produits (% en poids) | | | | | | |
| Ether diéthylique | 80,4 | 78,6 | 69,5 | 45,8 | 0,0 | 0,0 |
| Eau | 19,6 | 20,0 | 22,2 | 28,0 | 39,1 | 39,1 |
| Hydrocarbures | 0,0 | 1,4 | 8,3 | 26,2 | 60,9 | 60,9 |
| CO | 0,0 | 0,0 | 0,0 | 0,0 | 0,0 | 0,0 |
| $CO_2$ | 0,0 | 0,0 | 0,0 | 0,0 | 0,0 | 0,0 |
| Distribution des hydrocarbures (% en poids) | | | | | | |
| Méthane | 0,0 | 0,0 | 0,0 | 0,0 | 0,0 | 0,0 |
| Ethylène | 0,0 | 100,0 | 94,9 | 98,4 | 99,0 | 95,4 |
| Ethane | 0,0 | 0,0 | 0,0 | 0,0 | 0,0 | 0,0 |
| Propylène + propane | 0,0 | 0,0 | 0,3 | 0,1 | 0,5 | 0,6 |
| Butanes | 0,0 | 0,0 | 0,0 | 0,0 | 0,0 | 0,0 |
| Butènes | 0,0 | 0,0 | 4,8 | 1,5 | 0,5 | 0,3 |
| Pentanes | 0,0 | 0,0 | 0,0 | 0,0 | 0,0 | 0,0 |
| Pentènes | 0,0 | 0,0 | 0,0 | 0,0 | 0,0 | 0,0 |
| C5 + | 0,0 | 0,0 | 0,0 | 0,0 | 0,0 | 3.6 |
| % iso en C4 | 0,0 | 0,0 | 23,9 | 18,3 | 15,7 | 16,3 |
| % aromatiques en C5 + | 0,0 | 0,0 | 0,0 | 0,0 | 0,0 | 23,0 |

Ce tableau 9 révèle une sélectivité en carbone éthylénique de 99 % en poids en même temps qu'un taux de transformation de l'éthanol voisin de 100 % lorsqu'on opère à une température de 553 K (280 °C).

Exemple 17 : Préparation d'un catalyseur Na-ZSM-5 ayant un rapport molaire (M1 — M2/n)/(Si + M1) de 0,33 %

11,1 g de $SiO_2$ (aérosil) ont été ajoutés à une solution aqueuse d'hydroxyde de sodium formée de 1,6 g de NaOH dans 32 ml d'eau, de manière à obtenir une solution 1.

Cette solution 1 a été mélangée, en agitant vigoureusement, avec une solution 2 formée de 2,48 g de bromure de tétrapropylammonium dans 38 ml d'eau.

A la solution obtenue, on a ajouté, en agitant de manière continue, une troisième solution composée de 0,666 g d'aluminate de sodium dissous dans 10 ml d'eau.

Le pH du mélange a été réglé à une valeur de 10 à l'aide d'acide sulfurique concentré, après quoi on a ajouté 40 ml d'eau.

La composition du mélange réactionnel, sur une base molaire, était la suivante :

60 $SiO_2/Al_2O_3$ ; 4 NaOH ; 6 TPABr ; 666 $H_2O$.

Ce gel a été cristallisé dans des autoclaves sous la pression spontanée du mélange de réaction, pendant 3 jours, à une température de 423 K (150 °C) tout en agitant.

Le produit solide obtenu a été séparé par la solution liquide surnageante par filtration, puis lavé à l'eau, séché et calciné à l'air à une température de 823 K (550 °C) pendant 12 heures.

L'analyse aux rayonx X a montré que le produit était un zéolite ZSM-5 à 100 %.

L'échange ionique avec le cation sodium a été obtenu en chauffant le catalyseur au reflux dans un excès de chlorure de sodium 0,5 N, pendant 4 heures.

On a ainsi obtenu un catalyseur Na-ZSM-5 avec un rapport molaire (M1—M2/n)/(Si + M1) égal à 0,33 % sur base des résultats des analyses d'absorption atomique.

Exemple 18 : Transformation d'éthanol en éthylène à l'aide du catalyseur de l'exemple 17

On a fait passer, en phase gazeuse, une solution aqueuse d'éthanol sur le catalyseur de l'exemple 17 en opérant dans les mêmes conditions réactionnelles que dans l'exemple 2, si ce n'est que l'on en a modifié la vitesse spatiale horaire liquide (VSHL) et la durée de contact du réactif comme suit :

VSHL : 0,37 $heure^{-1}$

temps de contact : 0,9 seconde.

Les conditions opératoires et les analyses des produits sont données dans le tableau 10 suivant :

Tableau 10

Catalyseur : Na-ZSM-5 ((M1 — M2/n)/(Si + M1) % = 0,33)

| | | | | | |
|---|---|---|---|---|---|
| **Conditions de réaction** | | | | | |
| Température (K) | 554 | 575 | 593 | 616 | 635 |
| °C | 281 | 302 | 320 | 343 | 362 |
| VSHL (1/hr) | 0,37 | 0,37 | 0,37 | 0,37 | 0,37 |
| Transformation (%) | 50,3 | 80,3 | 100,0 | 100,0 | 100,0 |
| **Distribution des produits (% en poids)** | | | | | |
| Ether diéthylique | 45,1 | 3,6 | 0,0 | 0,0 | 0,0 |
| Acétaldéhyde | 0,0 | 0,0 | 0,0 | 0,0 | 0,8 |
| Eau | 28,1 | 38,2 | 39,1 | 39,1 | 38,8 |
| Hydrocarbures | 26,8 | 58,2 | 60,9 | 60,9 | 60,4 |
| CO | 0,0 | 0,0 | 0,0 | 0,0 | 0,0 |
| $CO_2$ | 0,0 | 0,0 | 0,0 | 0,0 | 0,0 |
| **Distribution des hydrocarbures (% en poids)** | | | | | |
| Méthane | 0,0 | 0,0 | 0,0 | 0,0 | 0,0 |
| Ethylène | 100,0 | 99,5 | 99,7 | 99,9 | 99,8 |
| Ethane | 0,0 | 0,0 | 0,0 | 0,0 | 0,0 |
| Propylène + propane | 0,0 | 0,0 | 0,0 | 0,1 | 0,2 |
| Butanes | 0,0 | 0,0 | 0,0 | 0,0 | 0,0 |
| Butènes | 0,0 | 0,5 | 0,3 | 0,0 | 0,0 |
| Pentanes | 0,0 | 0,0 | 0,0 | 0,0 | 0,0 |
| Pentènes | 0,0 | 0,0 | 0,0 | 0,0 | 0,0 |
| C5 + | 0,0 | 0,0 | 0,0 | 0,0 | 0,0 |
| % iso en C4 | 0,0 | 0,0 | 0,0 | 0,0 | 0,0 |
| % aromatiques en C5 + | 0,0 | 0,0 | 0,0 | 0,0 | 0,0 |

Ce tableau montre une sélectivité remarquable (au moins 99,7 % en poids) du catalyseur pour la formation d'éthylène et un taux de transformation de 100 % à partir d'une température de réaction de 593 K (320 °C).

Exemple 19 : Préparation d'un catalyseur Na-ZSM-5 ayant un rapport molaire (M1—M2/n)/(Si + M1) de 0,05 %

Ce catalyseur a été synthétisé de la manière décrite dans l'exemple 17.

L'échange ionique avec le cation sodium a été effectué, à trois reprises, de la manière décrite dans l'exemple 17.

Sur base des résultats d'absorption atomique, on a ainsi obtenu un catalyseur présentant un rapport molaire (M1—M2/n)/(Si + M1) de 0,05 %.

Exemple 20 : Transformation d'éthanol en éthylène à l'aide du catalyseur de l'exemple 19

On a opéré dans les mêmes conditions réactionnelles que dans l'exemple 2 en modifiant seulement la vitesse spatiale horaire liquide (VSHL) et la durée de contact du réactif comme suit :

VSHL : 0,10 $heure^{-1}$

temps de contact : 0,9 seconde.

Le tableau 11 suivant résume les résultats de cet essai.

Tableau 11

Catalyseur : Na-ZSM-5 ((M1—M2/n)/(Si + M1) % = 0,05)

| | | | | | | |
|---|---|---|---|---|---|---|
| **Conditions de réaction** | | | | | | |
| Température (K) | 629 | 642 | 652 | 662 | 668 | 707 |
| °C | 356 | 369 | 379 | 389 | 395 | 434 |
| VSHL (1/hr) | 0,10 | 0,10 | 0,10 | 0,10 | 0,10 | 0,10 |
| Transformation (%) | 77,7 | 92,9 | 97,3 | 100,0 | 100,0 | 100,0 |
| **Distribution des produits (% en poids)** | | | | | | |
| Ether diéthylique | 0,4 | 0,0 | 0,0 | 0,0 | 0,0 | 0,0 |
| Acétaldéhyde | 0,0 | 0,0 | 0,0 | 0,0 | 0,0 | 2,6 |
| Eau | 39,0 | 39,1 | 39,1 | 39,1 | 39,1 | 38,1 |
| Hydrocarbures | 60,6 | 60,9 | 60,9 | 60,9 | 69,9 | 59,3 |
| CO | 0,0 | 0,0 | 0,0 | 0,0 | 0,0 | 0,0 |
| $CO_2$ | 0,0 | 0,0 | 0,0 | 0,0 | 0,0 | 0,0 |
| **Distribution des hydrocarbures (% en poids)** | | | | | | |
| Méthane | 0,0 | 0,0 | 0,0 | 0,0 | 0,0 | 0,0 |
| Ethylène | 99,4 | 99,7 | 99,7 | 99,7 | 99,7 | 99,6 |
| Ethane | 0,0 | 0,0 | 0,0 | 0,0 | 0,0 | 0,0 |
| Propylène + propane | 0,3 | 0,3 | 0,3 | 0,3 | 0,3 | 0,4 |
| Butanes | 0,0 | 0,0 | 0,0 | 0,0 | 0,0 | 0,0 |
| Butènes | 0,3 | 0,0 | 0,0 | 0,0 | 0,0 | 0,0 |
| Pentanes | 0,0 | 0,0 | 0,0 | 0,0 | 0,0 | 0,0 |
| Pentènes | 0,0 | 0,0 | 0,0 | 0,0 | 0,0 | 0,0 |
| C5 + | 0,0 | 0,0 | 0,0 | 0,0 | 0,0 | 0,0 |
| % iso en C4 | 0,0 | 0,0 | 0,0 | 0,0 | 0,0 | 0,0 |
| % aromatiques en C5 + | 0,0 | 0,0 | 0,0 | 0,0 | 0,0 | 0,0 |

Ce tableau 11 révèle qu'on obtient un taux de transformation quantitatif en même temps qu'une sélectivité en carbone éthylénique au moins égale à 99 % en poids à partir d'une température de 662 K (389 °C) dans les conditions réactionnelles mentionnées.

Exemple 21 : Préparation d'un catalyseur Ca-ZSM-5 présentant un rapport molaire (M1—M2/n)/(Si + M1) de 0,24 %

Ce catalyseur a été synthétisé en utilisant les mêmes conditions opératoires que dans l'exemple 1, si ce n'est que l'on en a modifié la teneur en aluminium, en changeant la quantité d'aluminate de sodium

($NaAlO_2$) et que l'on a ajouté une quantité réglée de bromure de tétrapropylammonium (TPABr : 2,48 g) au lieu de l'hydroxyde.

La composition molaire du mélange de synthèse était la suivante :

0,5 ($R_2O$( ; 5 ($Na_2O$) ; 0,4 ($Al_2O_3$) ; 24 ($SiO_2$) ; 1 000 ($H_2O$)

dans laquelle R représente le cation organique tétrapropylammonium.

L'analyse par diffraction aux rayons X a révélé que le produit était constitué à 100 % de ZSM-5.

L'échange ionique avec le cation calcium a été effectué en chauffant le zéolite au reflux dans un excès de chlorure de calcium 0,5 N, pendant 4 heures.

Sur base des résultats d'absorption atomique, le rapport molaire (M1—M2/n)/(Si + M1) du catalyseur Ca-ZSM-5 était de 0,24 %. Ce catalyseur se présentait sous forme de cristaux d'une taille d'environ 4 microns.

Exemple 22 : Transformation d'éthanol en éthylène à l'aide du catalyseur de l'exemple 21

On a opéré dans les mêmes conditions que dans l'exemple 2, si ce n'est que l'on en a modifié la vitesse spatiale-horaire liquide (VSHL) et la durée de contact du réactif comme suit :

VSHL : 0,1 $heure^{-1}$

temps de contact : 0,9 seconde.

Le tableau 12 suivant indique les conditions opératoires et les produits obtenus à différentes températures de réaction.

Tableau 12

Catalyseur : Ca-ZSM-5 ((M1—M2/n)/(Si + M1) % = 0,24)

| | | | | | |
|---|---|---|---|---|---|
| Conditions de réaction | | | | | |
| Température (K) | 514 | 530 | 543 | 572 | 594 |
| °C | 241 | 257 | 270 | 299 | 321 |
| VSHL (1/hr) | 0,10 | 0,10 | 0,10 | 0,10 | 0,10 |
| Transformation (%) | 49,6 | 77,1 | 100,0 | 100,0 | 100,0 |
| Distribution des produits (% en poids) | | | | | |
| Ether diéthylique | 53,0 | 7,0 | 0,0 | 0,0 | 0,0 |
| Acétaldéhyde | 0,0 | 0,0 | 0,0 | 0,0 | 0,0 |
| Eau | 26,2 | 37,4 | 39,1 | 39,1 | 39,1 |
| Hydrocarbures | 20,8 | 55,6 | 60,9 | 60,9 | 60,9 |
| CO | 0,0 | 0,0 | 0,0 | 0,0 | 0,0 |
| $CO_2$ | 0,0 | 0,0 | 0,0 | 0,0 | 0,0 |
| Distribution des hydrocarbures (% en poids) | | | | | |
| Méthane | 0,0 | 0,0 | 0,0 | 0,0 | 0,0 |
| Ethylène | 100,0 | 99,8 | 99,7 | 99,8 | 99,7 |
| Ethane | 0,0 | 0,0 | 0,0 | 0,0 | 0,0 |
| Propylène + propane | 0,0 | 0,0 | 0,1 | 0,2 | 0,3 |
| Butanes | 0,0 | 0,0 | 0,0 | 0,0 | 0,0 |
| Butènes | 0,0 | 0,2 | 0,2 | 0,0 | 0,0 |
| Pentanes | 0,0 | 0,0 | 0,0 | 0,0 | 0,0 |
| Pentènes | 0,0 | 0,0 | 0,0 | 0,0 | 0,0 |
| C5 + | 0,0 | 0,0 | 0,0 | 0,0 | 0,0 |
| % iso en C4 | 0,0 | 0,0 | 0,0 | 0,0 | 0,0 |
| % aromatiques en C5 + | 0,0 | 0,0 | 0,0 | 0,0 | 0,0 |

Un taux de transformation de 100 % de même qu'une sélectivité en carbone éthylénique du catalyseur de plus de 99 % en poids ont été obtenus à partir d'une température de 543 K (270 °C).

Exemple 23 : Préparation d'un catalyseur H-ZSM-5 présentant un rapport molaire Al/Si + Al de 1,5 % après un traitement hydrothermique à l'aide de vapeur d'eau

On a préparé le catalyseur comme décrit dans l'exemple 17.

Sur base molaire, la composition du mélange de synthèse du zéolite ZSM-5 était la suivante :

60 $SiO_2/Al_2O_3$ ; 4 NaOH ; 6 TPABr ; 666 $H_2O$.

Après la synthèse, le produit solide a été activé en éliminant le cation organique TPA par calcination à l'air à 832 K (559 °C) pendant 12 heures.

Le catalyseur a été soumis à un prétraitement à l'aide de vapeur d'eau à une température de 973 K (700 °C) pendant 24 heures, en vue d'une diminution de rapport (M1—M2/n)/(Si + M1) qui était de 2,70 %, par désalumination partielle du réseau tridimensionnel rigide.

Le débit (VSHL en eau) auquel on a fait passer de l'eau en phase gazeuse au contact du catalyseur était de 0,8 heure$^{-1}$. L'échange ionique avec le cation ammonium a été effectué en chauffant le zéolite au reflux dans un excès de chlorure d'ammonium 0,5 N pendant 4 heures.

La forme hydrogène a été obtenue en calcinant le catalyseur dans le réacteur même à une température de 673 K (400 °C) sous un courant d'hélium pendant 1 heure.

Selon les résultats d'absorption atomique, après l'extraction de l'aluminium dans les positions cationiques, et selon l'analyse par R.M.N., on a ainsi obtenu un catalyseur H-ZSM-5 contenant 1,50 % de Al/Si + Al où Al est l'aluminium du réseau, donc lié par coordination en tétraèdres.

L'aluminium non lié par coordination tétraédrique a été lavé avec du NaOH de la manière décrite par Kerr (J. Catalysis, 15, 200 (1969)).

Exemple 24 : Transformation d'éthanol en éthylène à l'aide du catalyseur de l'exemple 23

Un échantillon du catalyseur de l'exemple 23 a été chargé dans un réacteur tubulaire. On a opéré dans les conditions réactionnelles décrites dans l'exemple 2, si ce n'est que l'on en a modifié la vitesse spatiale horaire liquide en éthanol et le temps de contact du réactif comme suit :

VSHL : 0,1 heure$^{-1}$

temps de contact : 0,9 seconde.

Le tableau 13 montre les conditions opératoires et les résultats.

Tableau 13

Catalyseur : H-ZSM-5 (Al/Si + Al % = 1,5)

| | | | | | | |
|---|---|---|---|---|---|---|
| **Conditions de réaction** | | | | | | |
| Température (K) | 490 | 500 | 510 | 529 | 550 | 576 |
| °C | 217 | 227 | 237 | 256 | 277 | 303 |
| VSHL (1/hr) | 0,10 | 0,10 | 0,10 | 0,10 | 0,10 | 0,10 |
| Transformation (%) | 60,8 | 77,4 | 100,0 | 100,0 | 100,0 | 100,0 |
| **Distribution des produits (% en poids)** | | | | | | |
| Ether diéthylique | 37,1 | 10,9 | 0,0 | 0,0 | 0,0 | 0,0 |
| Acétaldéhyde | 0,0 | 0,0 | 0,0 | 0,0 | 00,0 | 0,0 |
| Eau | 30,1 | 36,5 | 39,1 | 39,1 | 39,1 | 39,1 |
| Hydrocarbures | 32,8 | 52,6 | 60,9 | 60,9 | 60,9 | 60,9 |
| CO | 0,0 | 0,0 | 0,0 | 0,0 | 0,0 | 0,0 |
| $CO_2$ | 0,0 | 0,0 | 0,0 | 0,0 | 0,0 | 0,0 |
| **Distribution des hydrocarbures (% en poids)** | | | | | | |
| Méthane | 0,0 | 0,0 | 0,0 | 0,0 | 0,0 | 0,0 |
| Ethylène | 100,0 | 100,0 | 99,7 | 99,4 | 99,7 | 99,8 |
| Ethane | 0,0 | 0,0 | 0,0 | 0,0 | 0,0 | 0,0 |
| Propylène + propane | 0,0 | 0,0 | 0,3 | 0,6 | 0,3 | 0,2 |
| Butanes | 0,0 | 0,0 | 0,0 | 0,0 | 0,0 | 0,0 |
| Butènes | 0,0 | 0,0 | 0,0 | 0,0 | 0,0 | 0,0 |
| Pentanes | 0,0 | 0,0 | 0,0 | 0,0 | 0,0 | 0,0 |
| Pentènes | 0,0 | 0,0 | 0,0 | 0,0 | 0,0 | 0,0 |
| C5 + | 0,0 | 0,0 | 0,0 | 0,0 | 0,0 | 0,0 |
| % iso en C4 | 0,0 | 0,0 | 0,0 | 0,0 | 0,0 | 0,0 |
| % aromatiques en C5 + | 0,0 | 0,0 | 0,0 | 0,0 | 0,0 | 0,0 |

Lorsqu'on utilise comme catalyseur le zéolite H-ZSM-5 traité par de la vapeur d'eau, on peut obtenir avec un débit de 0,1 heure$^{-1}$ en éthanol d'une liqueur aqueuse contenant 90 % en poids d'éthanol, un taux de transformation de l'éthanol de 100 % et une sélectivité en carbone éthylénique de 99,7 % en poids en maintenant le catalyseur à une température de 510 K (237 °C).

Avec ce catalyseur H-ZSM-5, ayant le même rapport molaire Al/Si + Al % de départ (2,70 %) mais n'ayant pas subi de traitement hydrothermique, on ne réussit pas à atteindre un taux de transformation de 100 % en même temps qu'une sélectivité en carbone éthylénique d'au moins 99 % en poids, comme montré dans l'exemple 26 donné ci-après.

Exemple 25 (comparatif) : Préparation d'un catalyseur H-ZSM-5, ayant un rapport molaire Al/Si + Al de 2,70 %

Ce catalyseur a été préparé de la manière décrite dans l'exemple 17.

La composition molaire du mélange de synthèse du zéolite ZSM-5 était la suivante :

60 $SiO_2/Al_2O_3$ ; 4 NaOH ; 6 TPABr ; 666 $H_2O$

Le produit solide a été activé en éliminant le cation organique TPA à l'air à 823 K (550 °C) pendant 12 heures.

L'analyse aux rayons X a montré que le produit était du zéolite ZSM-5 à 100 %.

La forme hydrogène du zéolite a été obtenue par échange ionique comme décrit dans l'exemple 23.

On a ainsi obtenu un catalyseur H-ZSM-5 contenant 2,70 % de Al/Si + Al sous forme de cristaux d'environ 4 microns.

Exemple 26 (comparatif) : Transformation d'éthanol en éthylène à l'aide du catalyseur de l'exemple 25

On a exécuté cet essai comme décrit dans l'exemple 2, si ce n'est que l'on a modifié le VSHL et le temps de contact afin de chercher à optimaliser les conditions de réaction pour atteindre un taux de transformation de l'éthanol de 100 % et une sélectivité en carbone éthylénique au moins égale à environ 99 % en poids.

Le tableau suivant montre les conditions réactionnelles optimalisées et les résultats.

Tableau 14

Catalyseur : H-ZSM-5 (Al/Si + Al % = 2,70)

| | | | | | | |
|---|---|---|---|---|---|---|
| Conditions de réaction | | | | | | |
| Température (K) | 437 | 476 | 488 | 499 | 522 | 560 |
| °C | 164 | 203 | 215 | 226 | 249 | 287 |
| VSHL (1/hr) | 0,18 | 0,18 | 0,18 | 0,18 | 0,18 | 0,18 |
| Transformation (%) | 38,1 | 99,2 | 100,0 | 100,0 | 100,0 | 100,0 |
| Distribution des produits (% en poids) | | | | | | |
| Ether diéthylique | 66,2 | 0,0 | 0,0 | 0,0 | 0,0 | 0,0 |
| Acétaldéhyde | 0,0 | 0,0 | 0,0 | 0,0 | 0,0 | 0,0 |
| Eau | 23,0 | 39,1 | 39,1 | 39,0 | 39,1 | 39,2 |
| Hydrocarbures | 10,8 | 60,9 | 60,9 | 61,0 | 60,9 | 60,8 |
| CO | 0,0 | 0,0 | 0,0 | 0,0 | 0,0 | 0,0 |
| $CO_2$ | 0,0 | 0,0 | 0,0 | 0,0 | 0,0 | 0,0 |
| Distribution des hydrocarbures (% en poids) | | | | | | |
| Méthane | 0,0 | 0,0 | 0,0 | 0,0 | 0,0 | 0,0 |
| Ethylène | 98,6 | 92,1 | 85,9 | 72,8 | 49,1 | 27,0 |
| Ethane | 0,0 | 0,0 | 0,2 | 0,0 | 0,2 | 0,3 |
| Propylène + propane | 0,0 | 1,0 | 1,9 | 3,2 | 5,5 | 9,4 |
| Butanes | 0,0 | 0,4 | 1,5 | 2,4 | 2,9 | 8,0 |
| Butènes | 0,0 | 0,2 | 0,7 | 4,1 | 8,7 | 11,7 |
| Pentanes | 0,0 | 0,5 | 1,2 | 2,2 | 2,4 | 4,9 |
| Pentènes | 0,0 | 0,0 | 0,4 | 2,2 | 5,2 | 6,1 |
| C5 + | 1,4 | 5,8 | 8,2 | 13,1 | 26,0 | 32,6 |
| % iso en C4 | 0,0 | 23,0 | 39,0 | 59,5 | 61,2 | 68,9 |
| % aromatiques en C5 + | 0,0 | 0,0 | 8,4 | 10,0 | 35,4 | 59,6 |

Ce tableau montre que ce catalyseur dont le rapport molaire Al/Si + Al (%) est supérieur au maximum revendiqué, a une teneur en aluminium telle que son acidité est trop élevée pour qu'il puisse catalyser sélectivement la réaction de déshydratation d'éthanol en éthylène de façon à obtenir un taux de transformation quantitatif en même temps qu'une sélectivité en carbone éthylénique au moins égale à 99 % en poids.

Exemple 27 (comparatif) : Préparation d'un catalyseur Ca-ZSM-5 ayant un rapport molaire (M1—M2/n)/(Si + M1) de 1,65 %

Ce catalyseur a été préparé comme décrit dans l'exemple 17.

La composition du gel obtenu, sur base molaire, était la suivante :

60 $SiO_2/Al_2O_3$ ; 4 NaOH ; 6 TPABr ; 666 $H_2O$.

La calcination du produit solide, obtenu par cristallisation hydrothermique, a été réalisée de la manière décrite dans l'exemple 17.

Le zéolite a été ensuite soumis à un échange ionique à trois reprises en chauffant le catalyseur au reflux dans un excès de chlorure de calcium 0,5 N pendant 4 heures.

Sur base des résultats d'absorption atomique, le rapport molaire (M1—M2/n)/(Si + M1) du zéolite Ca-ZSM-5 était égal à 1,65 %.

Exemple 28 (comparatif) : Transformation d'éthanol en éthylène à l'aide du catalyseur de l'exemple 27

Cet essai a été exécuté de la manière décrite dans l'exemple 26.

Le tableau 15 suivant illustre les conditions réactionnelles et les résultats.

Tableau 15

Catalyseur : Ca-ZSM-5 ((M1—M2/n)/(Si + M1) % = 1,65)

| | | | | | | |
|---|---|---|---|---|---|---|
| **Conditions de réaction** | | | | | | |
| Température (K) | 489 | 511 | 523 | 543 | 573 | 613 |
| °C | 216 | 238 | 250 | 270 | 300 | 340 |
| VSHL (1/hr) | 0,18 | 0,18 | 0,18 | 0,18 | 0,18 | 0,18 |
| Transformation (%) | 61,1 | 76,6 | 100,0 | 100,0 | 100,0 | 100,0 |
| **Distribution des produits (% en poids)** | | | | | | |
| Ether diéthylique | 51,8 | 13,7 | 0,0 | 0,0 | 0,0 | 0,0 |
| Acétaldéhyde | 0,0 | 0,6 | 0,0 | 0,0 | 0,0 | 0,0 |
| Eau | 26,5 | 35,5 | 39,0 | 39,1 | 39,2 | 39,2 |
| Hydrocarbures | 21,7 | 50,2 | 61,0 | 60,9 | 60,8 | 60,8 |
| CO | 0,0 | 0,0 | 0,0 | 0,0 | 0,0 | 0,0 |
| $CO_2$ | 0,0 | 0,0 | 0,0 | 0,0 | 0,0 | 0,0 |
| **Distribution des hydrocarbures (% en poids)** | | | | | | |
| Méthane | 0,0 | 0,0 | 0,0 | 0,0 | 0,0 | 0,0 |
| Ethylène | 100,0 | 93,9 | 72,6 | 46,1 | 36,2 | 47,8 |
| Ethane | 0,0 | 0,0 | 0,8 | 0,4 | 0,4 | 0,0 |
| Propylène + propane | 0,0 | 0,4 | 3,3 | 8,1 | 10,6 | 13,6 |
| Butanes | 0,0 | 0,1 | 3,5 | 5,9 | 6,9 | 4,3 |
| Butènes | 0,0 | 1,1 | 4,0 | 12,3 | 12,9 | 13,2 |
| Pentanes | 0,0 | 1,3 | 3,1 | 3,8 | 3,5 | 1,9 |
| Pentènes | 0,0 | 0,0 | 1,8 | 6,9 | 6,0 | 5,1 |
| C5 + | 0,0 | 3,2 | 10,9 | 16,5 | 23,5 | 14,1 |
| % iso en C4 | 0,0 | 6,7 | 73,2 | 68,1 | 69,1 | 66,9 |
| % aromatiques en C5 + | 0,0 | 0,0 | 0,0 | 35,8 | 67,7 | 69,4 |

Il convient de noter que le rapport molaire (M1—M2/n)/(Si + M1) en % est supérieur au maximum revendiqué (1,5 %) et qu'il n'existe pas de conditions réactionnelles dans lesquelles le taux de transformation est de 100 % et la sélectivité en carbone éthylénique atteint plus de 99 % en poids.

Exemple 29 (comparatif) : Préparation d'un catalyseur La-ZSM-5 ayant un rapport molaire (M1—M2/n)/(Si + M1) de 1,75 %

On a synthétisé le catalyseur comme décrit dans l'exemple 17.

La composition du mélange de synthèse sur base molaire était :

60 $SiO_2/Al_2O_3$ ; 4 NaOH ; 6 TPABr ; 666 $H_2O$.

Après cristallisation, le produit solide a été séparé de la solution liquide, puis lavé à l'eau, séché et calciné à l'air.

Un échange ionique a été effectué à trois reprises en chauffant le catalyseur au reflux dans un excès de nitrate de lanthane ($La(NO_3)_3$) 0,1 N pendant 4 heures.

Le rapport molaire (M1—M2/n)/(Si + M1) du catalyseur ainsi obtenu était de 1,75 %.

Exemple 30 (comparatif) : Transformation d'éthanol en éthylène à l'aide du catalyseur de l'exemple 29

On a opéré de la manière décrite dans l'exemple 26.

Le tableau 16 résume les conditions opératoires et les résultats.

Tableau 16

Catalyseur : La-ZSM-5 ((M1—M2/n)/(Si + M1) % = 1,75)

| | | | | | | |
|---|---|---|---|---|---|---|
| **Conditions de réaction** | | | | | | |
| Température (K) | 476 | 500 | 510 | 519 | 541 | 562 |
| °C | 203 | 227 | 237 | 246 | 268 | 289 |
| VSHL (1/hr) | 0,18 | 0,18 | 0,18 | 0,18 | 0,18 | 0,18 |
| Transformation (%) | 60,2 | 76,4 | 100,0 | 100,0 | 100,0 | 100,0 |
| **Distribution des produits (% en poids)** | | | | | | |
| Ether diéthylique | 53,5 | 19,5 | 0,0 | 0,0 | 0,0 | 0,0 |
| Acétaldéhyde | 0,0 | 0,5 | 0,0 | 0,0 | 0,0 | 0,0 |
| Eau | 26,1 | 34,2 | 39,0 | 38,9 | 39,1 | 39,1 |
| Hydrocarbures | 20,4 | 45,8 | 61,0 | 61,1 | 60,9 | 60,9 |
| CO | 0,0 | 0,0 | 0,0 | 0,0 | 0,0 | 0,0 |
| $CO_2$ | 0,0 | 0,0 | 0,0 | 0,0 | 0,0 | 0,0 |
| **Distribution des hydrocarbures (% en poids)** | | | | | | |
| Méthane | 0,0 | 0,0 | 0,0 | 0,0 | 0,0 | 0,0 |
| Ethylène | 100,0 | 96,1 | 86,4 | 57,1 | 38,6 | 29,9 |
| Ethane | 0,0 | 0,0 | 0,7 | 1,1 | 0,5 | 0,4 |
| Propylène + propane | 0,0 | 0,4 | 2,3 | 4,9 | 8,7 | 10,8 |
| Butanes | 0,0 | 0,0 | 1,8 | 5,7 | 6,9 | 9,1 |
| Butènes | 0,0 | 1,3 | 1,3 | 6,9 | 13,2 | 12,9 |
| Pentanes | 0,0 | 0,2 | 1,8 | 4,7 | 4,7 | 5,0 |
| Pentènes | 0,0 | 0,0 | 0,2 | 3,8 | 7,6 | 6,7 |
| C5+ | 0,0 | 2,0 | 5,5 | 15,8 | 20,0 | 25,1 |
| % iso en C4 | 0,0 | 0,0 | 63,5 | 70,4 | 66,8 | 72,1 |
| % aromatiques en C5+ | 0,0 | 0,0 | 0,0 | 2,5 | 38,1 | 62,4 |

Ce catalyseur dont le rapport molaire (M1—M2/n)/(Si + M1) est supérieur à 1,5 % présente une acidité telle qu'il ne catalyse pas sélectivement la déshydratation d'éthanol en éthylène. En effet, un taux de transformation quantitatif en même temps qu'une sélectivité en carbone éthylénique d'au moins 99 % en poids ne peuvent pas être obtenus avec ce catalyseur.

Exemple 31 : Préparation d'un catalyseur H-ZSM-11 présentant un rapport molaire de Al/Si + Al de 1,0 %

On a synthétisé ce catalyseur en utilisant les mêmes conditions opératoires que dans l'exemple 1, si ce n'est que sa teneur en aluminium a été adaptée en ajoutant une quantité ajustée d'aluminate de sodium ($NaAlO_2$) dans le mélange réactionnel et que l'on a remplacé l'hydroxyde de tétrapropylammonium (TPAOH) par l'hydroxyde de tétrabutylammonium (TBAOH) en quantité réglée.

La composition molaire du mélange de synthèse du zéolite ZSM-11 était la suivante :

0,5 ($R_2O$) ; 5 ($NA_2O$) ; 0,12 ($Al_2O_3$) ; 24 ($SiO_2$) ; 1 000 ($H_2O$)

où R représente le cation tétrabutylammonium.

La calcination et l'échange ionique ont été réalisés de la manière décrite dans l'exemple 1.

L'analyse par diffraction aux rayons X a révélé la présence d'une phase fortement cristalline de ZSM-11.

Le catalyseur H-ZSM-11 obtenu contenait 1,0 % Al/Si + Al et se présentait sous forme de cristaux d'une taille d'environ 4 microns.

Exemple 32 : Transformation d'éthanol en éthylène à l'aide du catalyseur de l'exemple 31

On a opéré dans les mêmes conditions que dans l'exemple 2 en modifiant seulement la vitesse spatiale horaire liquide (VSHL) et la durée de contact du réactif comme suit :

VSHL : 0,15 $heure^{-1}$

temps de contact : 0,9 seconde.

Les résultats de cet essai et les conditions réactionnelles sont résumés dans le tableau 17 suivant :

Tableau 17

Catalyseur : H-ZSM-5 (Al/Si + Al % = 1,00)

| Conditions de réaction | | | | | | |
|---|---|---|---|---|---|---|
| Température (K) | 510 | 520 | 529 | 551 | 581 | 606 |
| °C | 237 | 247 | 256 | 278 | 308 | 333 |
| VSHL (1/hr) | 0,15 | 0,15 | 0,15 | 0,15 | 0,15 | 0,15 |
| Transformation (%) | 69,9 | 91,3 | 100,0 | 100,0 | 100,0 | 100,0 |
| **Distribution des produits (% en poids)** | | | | | | |
| Ether diéthylique | 16,0 | 1,8 | 0,0 | 0,0 | 0,0 | 0,0 |
| Acétaldéhyde | 0,0 | 0,2 | 0,0 | 0,0 | 0,0 | 0,0 |
| Eau | 35,2 | 38,6 | 39,1 | 39,1 | 39,1 | 39,1 |
| Hydrocarbures | 48,8 | 59,4 | 60,9 | 60,9 | 60,9 | 60,9 |
| CO | 0,0 | 0,0 | 0,0 | 0,0 | 0,0 | 0,0 |
| $CO_2$ | 0,0 | 0,0 | 0,0 | 0,0 | 0,0 | 0,0 |
| **Distribution des hydrocarbures (% en poids)** | | | | | | |
| Méthane | 0,0 | 0,0 | 0,0 | 0,0 | 0,0 | 0,0 |
| Ethylène | 100,0 | 99,2 | 99,0 | 99,0 | 99,3 | 99,3 |
| Ethane | 0,0 | 0,0 | 0,0 | 0,0 | 0,0 | 0,0 |
| Propylène + propane | 0,0 | 0,2 | 0,4 | 0,5 | 0,4 | 0,4 |
| Butanes | 0,0 | 0,1 | 0,1 | 0,1 | 0,0 | 0,0 |
| Butènes | 0,0 | 0,5 | 0,5 | 0,4 | 0,3 | 0,3 |
| Pentanes | 0,0 | 0,0 | 0,0 | 0,0 | 0,0 | 0,0 |
| Pentènes | 0,0 | 0,0 | 0,0 | 0,0 | 0,0 | 0,0 |
| C5+ | 0,0 | 0,0 | 0,0 | 0,0 | 0,0 | 0,0 |
| % iso en C4 | 0,0 | 12,1 | 21,5 | 20,9 | 13,9 | 8,7 |
| % aromatiques en C5+ | 0,0 | 0,0 | 0,0 | 0,0 | 0,0 | 0,0 |

Une sélectivité en carbone éthylénique au moins égale à environ 99 % en poids, en même temps qu'un taux de transformation quantitatif se sont manifestés à partir d'une température de 529 K (256 °C).

Exemple 33 : Préparation d'un catalyseur H-ZSM-48 présentant un rapport molaire (M1 — M2/n)/(Si + M1) = 0,24 %

Les solutions de départ suivantes ont été utilisées :

a) 37,34 g d'une solution aqueuse de silicate de sodium contenant de l'aluminium à titre d'impureté (dans 80 ml d'eau).

b) 1,87 ml d'acide sulfurique concentré.

c) 8,434 g de bromure de tétraméthylammonium (TMABr) et 36,2 g d'octylamine dans 80 ml d'eau.

L'acide sulfurique (b) a été ajouté à la solution (a) en agitant vigoureusement. Cette solution a été mélangée avec la solution (c). La composition du gel obtenu, sur base molaire, était la suivante :

600 $SiO_2/Al_2O_3$ ; 5 $Na_2O$ ; 5,5 TMABr ; 27,5 octylamine ; 1 000 $H_2O$.

Ce gel a été agité dans des autoclaves à une température de 433 K (160 °C) pendant 2 jours. Après lavage, séchage et calcination à l'air à une température de 823 K (550 °C) pendant 12 heures, le produit solide a été analysé par diffraction aux rayons X. L'analyse a révélé la présence d'une phase fortement cristalline de ZSM-48.

Le zéolite a été mis en forme H de la manière décrite dans l'exemple 1.

Sur base des résultats d'absorption atomique, le rapport (M1 — M2/n)/(Si + M1) du zéolite était de 0,24 %.

Exemple 34 : Transformation d'éthanol en éthylène à l'aide d'un catalyseur de l'exemple 33

On a opéré dans les mêmes conditions réactionnelles que dans l'exemple 2 en modifiant seulement la vitesse spatiale horaire liquide (VSHL) et la durée de contact du réactif comme suit :

VSHL : 0,27 heure$^{-1}$

temps de contact : 0,9 seconde.

Le tableau 18 résume les résultats de cet essai.

Tableau 18

Catalyseur : H-ZSM-48 (M1 — M2/n)/(Si + M1) % = 0,24

| | | | | | |
|---|---|---|---|---|---|
| Conditions de réaction | | | | | |
| Température (K) | 563 | 601 | 621 | 644 | 673 |
| °C | 290 | 328 | 348 | 371 | 400 |
| VSHL (1/hr) | 0,27 | 0,27 | 0,27 | 0,27 | 0,27 |
| Transformation (%) | 60,3 | 100,0 | 100,0 | 100,0 | 100,0 |
| Distribution des produits (% en poids) | | | | | |
| Ether diéthylique | 12,0 | 0,0 | 0,0 | 0,0 | 0,0 |
| Acétaldéhyde | 0,1 | 0,2 | 0,3 | 0,4 | 0,8 |
| Eau | 36,2 | 39,0 | 39,0 | 39,0 | 38,8 |
| Hydrocarbures | 51,7 | 60,8 | 60,7 | 60,6 | 60,4 |
| CO | 0,0 | 0,0 | 0,0 | 0,0 | 0,0 |
| $CO_2$ | 0,0 | 0,0 | 0,0 | 0,0 | 0,0 |
| Distribution des hydrocarbures (% en poids) | | | | | |
| Méthane | 0,0 | 0,0 | 0,0 | 0,0 | 0,0 |
| Ethylène | 99,1 | 99,3 | 99,5 | 99,7 | 99,7 |
| Ethane | 0,0 | 0,0 | 0,0 | 0,0 | 0,0 |
| Propylène + propane | 0,1 | 0,0 | 0,0 | 0,0 | 0,1 |
| Butanes | 0,0 | 0,0 | 0,0 | 0,0 | 0,0 |
| Butènes | 0,8 | 0,7 | 0,5 | 0,3 | 0,2 |
| Pentanes | 0,0 | 0,0 | 0,0 | 0,0 | 0,0 |
| Pentènes | 0,0 | 0,0 | 0,0 | 0,0 | 0,0 |
| C5 + | 0,0 | 0,0 | 0,0 | 0,0 | 0,0 |
| % iso en C4 | 0,0 | 0,0 | 0,0 | 0,0 | 0,0 |
| % aromatiques en C5 + | 0,0 | 0,0 | 0,0 | 0,0 | 0,0 |

Ce tableau révèle une sélectivité en carbone éthylénique voisin de 100 % en poids en même temps qu'un taux de transformation quantitatif à une température de réaction de 601 K (328 °C).

Exemple 35 : Préparation d'un catalyseur H-ZSM-5 (Fe) ayant un rapport molaire (M1 — M2/n)/(Si + M1) de 0,01 %

On a synthétisé ce catalyseur de la manière décrite dans l'exemple 3, si ce n'est que l'on a ajouté au lieu de nitrate de l'aluminium (Al $(NO_3)_3 \cdot 9H_2O$) du nitrate de fer (Fe $(NO_3)_3 \cdot 9H_2O$) en quantité réglée dans le mélange de synthèse.

La composition molaire du produit était la suivante :

100 $SiO_2/Fe(NO_3)_3$ ; 13 NaOH ; 83 TPABr ; 13 $NH_4OH$ ; 662 $C_3H_5$ $(OH)_3$.

Le produit solide obtenu a été calciné à l'air à 832 K (559 °C) pendant 12 heures.

Le zéolite obtenu a été ensuite soumis à un échange ionique en chauffant le catalyseur au reflux dans un excès de chlorure d'ammonium 0,5 N pendant 4 heures.

La forme hydrogène a été obtenue en calcinant le catalyseur dans le réacteur même à une température de 673 K (400 °C) sous un courant d'hélium pendant 1 heure.

Suivant les résultats d'absorption atomique, le rapport Si/Fe où le fer est lié par coordination en tétraèdres, précipité ou sous forme cationique était de 100.

Selon l'analyse par l'absorption atomique, ce zéolite ne contenait pas d'aluminium comme impureté.

La teneur en fer incorporée au réseau tridimensionnel du zéolite a été déterminée par R.P.E. après la synthèse et le prétraitement.

Le rapport $Fe_O/Fe_T$, où $Fe_O$ est la quantité de fer non liée par coordination en tétraèdres et où $Fe_T$ est la quantité de fer incorporée au réseau, a été estimé par l'intégration du signal R.P.E., le rapport Si/Fe initial étant fixé par l'analyse par absorption atomique.

Le rapport $Si/Fe_T$ a été déterminé par la méthode et les résultats décrits par Derouane, E.G. (Proceedings of Third International Conference on Molecular Sieves, « Molecular Sieves » Sept. 3-7, 1973, Ed. Uytterhoeven, p. 337-342).

Le rapport molaire (M1 — M2/n)/(Si + M1) était de 0,01 %.

Exemple 36 : Transformation d'éthanol en éthylène à l'aide du catalyseur de l'exemple 35

On a opéré dans les mêmes conditions réactionnelles que dans l'exemple 2 en modifiant seulement la

vitesse spatiale horaire liquide (VSHL) et la durée de contact du réactif comme suit :

VSHL : 0,15 heure$^{-1}$

temps de contact : 0,9 seconde.

Les résultats de cet essai figurent au tableau 19.

Ce tableau montre qu'un taux de transformation de 100 % en même temps qu'une sélectivité en carbone éthylénique d'au moins 99 % en poids ont été obtenus à partir d'une température de 538 K (265 °C).

Tableau 19

Catalyseur : H-ZSM-5 (Fe) ((M1 — M2/n)/(Si + M1) % = 0,01)

| | | | | | | |
|---|---|---|---|---|---|---|
| **Conditions de réaction** | | | | | | |
| Température (K) | 517 | 527 | 538 | 560 | 569 | 589 |
| °C | 244 | 254 | 265 | 287 | 296 | 316 |
| VSHL (1/hr) | 0,15 | 0,15 | 0,15 | 0,15 | 0,15 | 0,15 |
| Transformation (%) | 67,6 | 87,4 | 100,0 | 100,0 | 100,0 | 100,0 |
| **Distribution des produits (% en poids)** | | | | | | |
| Ether diéthylique | 13,9 | 1,7 | 0,0 | 0,0 | 0,0 | 0,0 |
| Acétaldéhyde | 0,3 | 0,2 | 0,0 | 0,0 | 0,0 | 0,2 |
| Eau | 35,6 | 38,6 | 39,1 | 39,1 | 39,1 | 39,0 |
| Hydrocarbures | 50,2 | 59,5 | 60,9 | 60,9 | 60,9 | 60,8 |
| CO | 0,0 | 0,0 | 0,0 | 0,0 | 0,0 | 0,0 |
| $CO_2$ | 0,0 | 0,0 | 0,0 | 0,0 | 0,0 | 0,0 |
| **Distribution des hydrocarbures (% en poids)** | | | | | | |
| Méthane | 0,0 | 0,0 | 0,0 | 0,0 | 0,0 | 0,0 |
| Ethylène | 100,0 | 99,5 | 99,3 | 99,6 | 99,7 | 99,7 |
| Ethane | 0,0 | 0,0 | 0,0 | 0,0 | 0,0 | 0,0 |
| Propylène + propane | 0,0 | 0,1 | 0,2 | 0,1 | 0,1 | 0,1 |
| Butanes | 0,0 | 0,0 | 0,0 | 0,0 | 0,0 | 0,0 |
| Butènes | 0,0 | 0,4 | 0,5 | 0,3 | 0,2 | 0,2 |
| Pentanes | 0,0 | 0,0 | 0,0 | 0,0 | 0,0 | 0,0 |
| Pentènes | 0,0 | 0,0 | 0,0 | 0,0 | 0,0 | 0,0 |
| C5 + | 0,0 | 0,0 | 0,0 | 0,0 | 0,0 | 0,0 |
| % iso en C4 | 0,0 | 0,0 | 0,0 | 0,0 | 0,0 | 0,0 |
| % aromatiques en C5 + | 0,0 | 0,0 | 0,0 | 0,0 | 0,0 | 0,0 |

Exemple 37 : Préparation d'un catalyseur H-Na-Y ayant un rapport molaire (M1 — M2/n)/(Si + M1) de 1,15 %

Le catalyseur a été préparé à partir d'un zéolite du commerce du type Faujasite : NaY (Ventron, Si/Al = 2,52).

Le zéolite a été calciné et soumis à un échange ionique partiel avec le cation ammonium en chauffant le catalyseur au reflux dans une solution de chlorure d'ammonium $11,4 \cdot 10^{-3}$ (100 ml/g catalyseur) pendant 4 heures.

La forme hydrogène a été obtenue en calcinant ce catalyseur $NH_4$-Na-Y dans le réacteur même à une température de 673 K (400 °C) sous un courant d'hélium pendant 1 heure.

Selon les résultats d'absorption atomique, le rapport molaire (M1 — M2/n)/(Si + M1) du zéolite ainsi obtenu était égal à 1,15 %.

Exemple 38 : Transformation d'éthanol en éthylène à l'aide du catalyseur de l'exemple 37

Un échantillon du catalyseur de l'exemple 37 a été chargé dans un réacteur tubulaire. On a opéré dans les conditions réactionnelles décrites dans l'exemple 2, si ce n'est que l'on en a modifié la vitesse spatiale horaire liquide en éthanol et le temps de contact du réactif comme suit :

VSHL : 0,1 heure$^{-1}$

temps de contact : 0,9 seconde.

Le tableau 20 montre les conditions opératoires et les résultats.

Ce tableau révèle qu'on obtient une sélectivité en carbone éthylénique égale à 99 % en poids en même temps qu'un taux de transformation quantitatif à partir de 521 K (248 °C) dans les conditions réactionnelles mentionnées.

Tableau 20

Catalyseur : H-Na-Y ((M1—M2/n)/(Si + M1) % = 1,15)

| | | | | | |
|---|---|---|---|---|---|
| **Conditions de réaction** | | | | | |
| Température (K) | 494 | 512 | 521 | 529 | 545 |
| °C | 221 | 239 | 248 | 256 | 272 |
| VSHL (1/hr) | 0,10 | 0,10 | 0,10 | 0,10 | 0,10 |
| Transformation (%) | 71,6 | 79,8 | 100,0 | 100,0 | 100,0 |
| **Distribution des produits (% en poids)** | | | | | |
| Ether diéthylique | 37,4 | 21,3 | 0,0 | 0,0 | 0,0 |
| Acétaldéhyde | 0,0 | 0,0 | 0,0 | 0,0 | 0,0 |
| Eau | 30,0 | 33,9 | 39,1 | 39,1 | 39,1 |
| Hydrocarbures | 32,6 | 44,7 | 60,9 | 60,9 | 60,9 |
| CO | 0,0 | 0,0 | 0,0 | 0,0 | 0,0 |
| $CO_2$ | 0,0 | 0,0 | 0,0 | 0,0 | 0,0 |
| **Distribution des hydrocarbures (% en poids)** | | | | | |
| Méthane | 0,0 | 0,0 | 0,0 | 0,0 | 0,0 |
| Ethylène | 100,0 | 100,0 | 99,5 | 99,6 | 99,6 |
| Ethane | 0,0 | 0,0 | 0,0 | 0,0 | 0,0 |
| Propylène + propane | 0,0 | 0,0 | 0,0 | 0,0 | 0,0 |
| Butanes | 0,0 | 0,0 | 0,2 | 0,2 | 0,2 |
| Butènes | 0,0 | 0,0 | 0,3 | 0,2 | 0,2 |
| Pentanes | 0,0 | 0,0 | 0,0 | 0,0 | 0,0 |
| Pentènes | 0,0 | 0,0 | 0,0 | 0,0 | 0,0 |
| C5+ | 0,0 | 0,0 | 0,0 | 0,0 | 0,0 |
| % iso en C4 | 0,0 | 0,0 | 40,5 | 44,4 | 43,8 |
| % aromatiques en C5+ | 0,0 | 0,0 | 0,0 | 0,0 | 0,0 |

Exemple 39 : Préparation d'un catalyseur H-Mordénite présentant un rapport molaire (M1 — M2/n)/(Si + M1) de 0,01 %

Ce catalyseur a été préparé à partir d'un zéolite du commerce : Na-Mordénite (Norton Zeolon 100).

15 g du zéolite Na-Mordénite ont été ajoutés à de l'acide nitrique 4N en utilisant un rapport liquide/solide de 4. Le mélange a été agité pendant 8 heures à la température ambiante.

Par filtration, on a séparé le zéolite de la solution, puis on a lavé à l'eau et séché à l'air à une température de 343 K.

On a ajouté une deuxième fois de l'acide nitrique 4N au zéolite en utilisant le même rapport liquide/solide de 4.

Après avoir chauffé ce mélange jusqu'à 323 K (50 °C), on l'a agité à cette température pendant 24 heures. Puis, le produit solide a été séparé de la solution.

Le zéolite a été soumis ensuite à une calcination à « deep bed » à une température de 853 K (580 °C) pendant 16 heures.

Après cette calcination, on a répété le traitement avec l'acide nitrique, si ce n'est que le rapport liquide/solide était de 50 et que l'on a chauffé le mélange au reflux pendant 24 heures.

Le produit solide a été séparé de la solution, lavé à l'eau et séché.

Ensuite, le zéolite a été activé en le calcinant dans le réacteur à 673 K (400 °C) sous un courant d'hélium pendant 1 heure.

Ainsi, on a obtenu un catalyseur H-Mordénite présentant un rapport molaire (M1 — M2/n)/(Si + M1) de 0,01 %.

Exemple 40 : Transformation d'éthanol en éthylène à l'aide du catalyseur de l'exemple 39

On a exécuté cet essai comme décrit dans l'exemple 2, si ce n'est que l'on en a modifié la vitesse spatiale horaire liquide en éthanol et le temps de contact du réactif comme suit :

VSHL : 0,07 $heure^{-1}$

temps de contact : 0,9 seconde.

Le tableau 21 montre les conditions réactionnelles optimalisées et les résultats.

Ce tableau montre que pour ce catalyseur, une sélectivité en carbone éthylénique au moins égale à environ 99 % en poids en même temps qu'un taux de transformation quantitatif ont été obtenus à une température de 722 K (449 °C).

Tableau 21

Catalyseur : H-Mordénite ((M1 — M2/n)/(Si + M1) % = 0,01)

| | | | | | | |
|---|---|---|---|---|---|---|
| **Conditions de réaction** | | | | | | |
| Température (K) | 588 | 657 | 688 | 698 | 717 | 722 |
| °C | 315 | 384 | 415 | 425 | 444 | 449 |
| VSHL (1/hr) | 0,07 | 0,07 | 0,07 | 0,07 | 0,07 | 0,07 |
| Transformation (%) | 10,6 | 43,7 | 76,60 | 95,6 | 97,9 | 100,0 |
| **Distribution des produits (% en poids)** | | | | | | |
| Ether diéthylique | 13,6 | 0,0 | 0,0 | 0,0 | 0,0 | 0,0 |
| Acétaldéhyde | 0,0 | 0,0 | 0,0 | 0,0 | 0,0 | 0,0 |
| Eau | 35,8 | 39,1 | 39,1 | 39,1 | 39,1 | 39,1 |
| Hydrocarbures | 50,6 | 60,9 | 60,9 | 60,9 | 60,9 | 60,9 |
| CO | 0,0 | 0,0 | 0,0 | 0,0 | 0,0 | 0,0 |
| $CO_2$ | 0,0 | 0,0 | 0,0 | 0,0 | 0,0 | 0,0 |
| **Distribution des hydrocarbures (% en poids)** | | | | | | |
| Méthane | 0,0 | 0,0 | 0,0 | 0,1 | 0,3 | 0,3 |
| Ethylène | 100,0 | 100,0 | 100,0 | 99,6 | 99,1 | 99,1 |
| Ethane | 0,0 | 0,0 | 0,0 | 0,0 | 0,0 | 0,0 |
| Propylène + propane | 0,0 | 0,0 | 0,0 | 0,3 | 0,6 | 0,6 |
| Butanes | 0,0 | 0,0 | 0,0 | 0,0 | 0,0 | 0,0 |
| Butènes | 0,0 | 0,0 | 0,0 | 0,0 | 0,0 | 0,0 |
| Pentanes | 0,0 | 0,0 | 0,0 | 0,0 | 0,0 | 0,0 |
| Pentènes | 0,0 | 0,0 | 0,0 | 0,0 | 0,0 | 0,0 |
| C5 + | 0,0 | 0,0 | 0,0 | 0,0 | 0,0 | 0,0 |
| % iso en C4 | 0,0 | 0,0 | 0,0 | 0,0 | 0,0 | 0,0 |
| % aromatiques en C5 + | 0,0 | 0,0 | 0,0 | 0,0 | 0,0 | 0,0 |

Exemple 41 : Préparation d'un catalyseur La-ZSM-5 présentant un rapport (M1 — M2/n)/(Si + M1) de 1,5 %

Ce catalyseur a été préparé comme décrit dans l'exemple 17.

La composition du mélange de synthèse, sur base molaire était :

60 $SiO_2/Al_2O_3$ ; 4 NaOH ; 6 TPABr ; 666 $H_2O$.

Après la cristallisation hydrothermique, le produit solide a été séparé de la solution, puis lavé à l'eau, séché et calciné à l'air, de la manière décrite dans l'exemple 17.

Le zéolite a été ensuite ajouté à une solution d'ammoniaque (pH = 8). Le mélange a été agité à la température ambiante pendant quelques minutes, après quoi on a séparé le produit solide de la solution et on l'a séché.

Un échange ionique a été effectué, à trois reprises, en chauffant le catalyseur au reflux dans un excès de nitrate de lanthane ($La(NO_3)_3$) 0,1 N pendant 4 heures.

Puis le zéolite a été soumis à un échange ionique avec le cation sodium en chauffant le catalyseur au reflux dans une solution de chlorure de sodium $16 \cdot 10^{-4}$ N (100 ml/g catalyseur) pendant 4 heures.

Sur base des résultats d'absorption atomique, le rapport molaire (M1 — M2/n)/(Si + M1) du zéolite ainsi obtenu était égal à 1,5 %.

Exemple 42 : Transformation d'éthanol en éthylène à l'aide du catalyseur de l'exemple 41

On a opéré dans les mêmes conditions que dans l'exemple 2 en modifiant seulement la vitesse spatiale horaire liquide (VSHL) et la durée de contact du réactif comme suit :

VSHL : 0,07 $heure^{-1}$

temps de contact : 0,9 seconde.

Les résultats de cet essai et les conditions réactionnelles sont résumés dans le tableau 22.

Un taux de transformation de 100 % en même temps qu'une sélectivité en carbone éthylénique au moins égale à 99 % en poids ont été obtenus à une température de réaction d'environ 498 K (225 °C) dans les conditions réactionnelles mentionnées.

Tableau 22

Catalyseur : LA-ZSM-5 ((M1 — M1/n)/(Si + M1) % = 1,5)

| | | | | | | |
|---|---|---|---|---|---|---|
| **Conditions de réaction** | | | | | | |
| Température (K) | 408 | 444 | 487 | 490 | 498 | 510 |
| °C | 135 | 171 | 214 | 217 | 225 | 237 |
| VSHL (1/hr) | 0,07 | 0,07 | 0,07 | 0,07 | 0,07 | 0,07 |
| Transformation (%) | 15,0 | 35,4 | 94,1 | 97,6 | 100,0 | 100,0 |
| **Distribution des produits (% en poids)** | | | | | | |
| Ether diéthylique | 78,6 | 52,8 | 0,3 | 0,0 | 0,0 | 0,0 |
| Acétaldéhyde | 0,0 | 0,0 | 0,0 | 0,0 | 0,0 | 0,0 |
| Eau | 20,0 | 26,3 | 39,0 | 39,1 | 39,1 | 39,1 |
| Hydrocarbures | 1,4 | 20,9 | 60,7 | 60,9 | 60,9 | 60,9 |
| CO | 0,0 | 0,0 | 0,0 | 0,0 | 0,0 | 0,0 |
| $CO_2$ | 0,0 | 0,0 | 0,0 | 0,0 | 0,0 | 0,0 |
| **Distribution des hydrocarbures (% en poids)** | | | | | | |
| Méthane | 0,0 | 0,0 | 0,0 | 0,0 | 0,0 | 0,0 |
| Ethylène | 100,0 | 100,0 | 100,0 | 99,7 | 99,5 | 98,5 |
| Ethane | 0,0 | 0,0 | 0,0 | 0,0 | 0,0 | 0,0 |
| Propylène + propane | 0,0 | 0,0 | 0,0 | 0,3 | 0,5 | 0,8 |
| Butanes | 0,0 | 0,0 | 0,0 | 0,0 | 0,0 | 0,4 |
| Butènes | 0,0 | 0,0 | 0,0 | 0,0 | 0,0 | 0,3 |
| Pentanes | 0,0 | 0,0 | 0,0 | 0,0 | 0,0 | 0,0 |
| Pentènes | 0,0 | 0,0 | 0,0 | 0,0 | 0,0 | 0,0 |
| C5 + | 0,0 | 0,0 | 0,0 | 0,0 | 0,0 | 0,0 |
| % iso en C4 | 36,8 | 0,0 | 0,0 | 0,0 | 0,0 | 59,8 |
| % aromatiques en C5 + | 0,0 | 0,0 | 0,0 | 0,0 | 0,0 | 0,0 |

## Revendications

1. Procédé d'obtention d'éthylène à partir d'éthanol anhydre ou aqueux à l'aide d'un catalyseur du type zéolite contenant un silicate de métal M1 ayant une valence égale à 3 et une coordination tétraédrique et contenant éventuellement un autre métal M2 compensateur de charge choisi parmi les métaux alcalins et alcalino-terreux et les lanthanides, caractérisé en ce qu'on met de l'éthanol anhydre ou aqueux en contact avec un catalyseur du type précité contenant une proportion du métal M1 telle que le rapport molaire

$$\frac{M1 - M2/n}{Si + M1}$$

(où n est la valence dudit autre métal) en pourcents est au maximum d'environ 1,5 à une température comprise entre 400 et 800 K (127 et 527 °C) et à un débit de 0,05 à 10 heures$^{-1}$, de telle sorte que le taux de transformation de l'éthanol soit voisin de 100 % et que la sélectivité en carbone éthylénique soit au moins égale à environ 99 % en poids.

2. Procédé suivant la revendication 1, caractérisé en ce qu'on utilise un catalyseur dans lequel le métal M1 est de l'aluminium, du bore, du béryllium, du chrome ou du fer ou un mélange d'aluminium et d'au moins un autre de ces métaux.

3. Procédé suivant la revendication 2, caractérisé en ce qu'on utilise un catalyseur contenant de l'aluminium comme métal M1.

4. Procédé suivant la revendication 2, caractérisé en ce qu'on utilise un catalyseur exempt de métal M2.

5. Procédé suivant la revendication 1, caractérisé en ce qu'on utilise un catalyseur dans lequel le rapport molaire

$$\frac{M1 - M2/n}{Si + M1}$$

est compris entre 0,01 et 1 %.

6. Procédé suivant la revendication 1, caractérisé en ce qu'on utilise un catalyseur de type ZSM-5, de préférence sous forme hydrogène H-ZSM-5.

7. Procédé suivant l'une quelconque des revendications précédentes caractérisé en ce qu'on fait réagir avec le catalyseur une liqueur éthanolique contenant au moins environ 4 % en poids d'éthanol.

8. Procédé suivant la revendication 7, caractérisé en ce que la liqueur éthanolique est une liqueur de fermentation d'une biomasse ou d'un produit d'un gaz de synthèse dérivé de charbon ou de pétrole contenant de l'éthanol.

**Claims**

1. Process for obtaining ethylene from anhydrous or aqueous ethanol by means of a catalyst of the zeolite type containing a silicate of a metal M1 having a valence equal to 3 and a tetraedric coordination and containing possibly another charge compensating metal M2 selected among the alkaline and alkaline-earth metals and the lanthanides, characterized in that anhydrous or aqueous ethanol is contacted with a catalyst of said type containing such a proportion of metal M1 that the molar ratio

$$\frac{M1 - M2/n}{Si + M1}$$

(where n is the valence of said other metal) in percents is at most equal to about 1.5 at a temperature comprised between 400 and 800 K (127 and 527 °C) and at a flow rate from 0.05 to 10 hours$^{-1}$, so that the conversion rate of the ethanol is almost 100 % and that the ethylene carbon selectivity is at least equal to about 99 % by weight.

2. Process according to claim 1, characterized in that a catalyst in which the metal M1 is aluminium, boron, beryllium, chromium, iron or a mixture of aluminium and at aleast another of these metals is used.

3. Process according to claim 2, characterized in that a catalyst containing aluminium as metal M1 is used.

4. Process according to claim 2, characterized in that a catalyst free from metal M2 is used.

5. Process according to claim 1, characterized in that a catalyst in which the molar ratio

$$\frac{M1 - M2/n}{Si + M1}$$

is comprised between 0.01 and 1 % is used.

6. Process according to claim 1, characterized in that a catalyst of the ZSM-5 type, preferably in hydrogen form H-ZSM-5, is used.

7. Process according to anyone of the preceding claims, characterized in that a liquor containing at least 4 % by weight of ethanol is reacted on the catalyst.

8. Process according to claim 7, characterized in that the ethanolic liquor is a liquor of fermentation of biomass or of a product of synthesis gas derived from coal or petroleum containing ethanol.

**Patentansprüche**

1. Verfahren zur Herstellung von Ethylen, ausgehend von wasserfreiem oder wässrigem Ethanol mit Hilfe eines Katalysators vom Zeolith-Typ, enthaltend ein Silicat eines Metalls M1 mit einer Wertigkeit von 3 und einer tetraedischen Koordination und enthaltend gegebenenfalls ein anderes Metall M2 zum Ladungsausgleich, ausgewählt aus Alkali- und Erdalkalimetallen und Lanthaniden, dadurch gekennzeichnet, daß man das wasserfreie oder wässrige Ethanol mit einem Katalysator vom oben genanntem Typ, enthaltend einen Anteil Metall M1, so daß das Molverhältnis

$$\frac{M1 - M2/n}{Si + M1}$$

(wobei n die Wertigkeit des erwähnten anderen Metalls ist) in Prozent maximal etwa 1,5 beträgt bei einer Temperatur zwischen 400 und 800 K (127 bis 527 °C) in Kontakt bringt mit einem Durchsatz von 0,05 bis 10 h$^{-1}$, so daß der Umwandlungsgrad des Ethanols nahe bei 100 % liegt und daß die Selektivität für Ethylen mindestens etwa 99 Gew.-% beträgt.

2. Verfahren nach Anspruch 1, dadurch gekennzeichnet, daß man einen Katalysator verwendet, bei dem das Metall M1 Aluminium, Bor, Beryllium, Chrom oder Eisen oder ein Gemisch aus Aluminium und mindestens einem der anderen Metalle ist.

3. Verfahren nach Anspruch 2, dadurch gekennzeichnet, daß man einen Katalysator verwendet, der Aluminium als Metall M1 enthält.

4. Verfahren nach Anspruch 2, dadurch gekennzeichnet, daß man einen Katalysator verwendet, der kein Metall M2 enthält.

5. Verfahren nach Anspruch 1, dadurch gekennzeichnet, daß man einen Katalysator verwendet, in dem das Molverhältnis

$$\frac{M1 - M2/n}{Si + M1}$$

zwischen 0,01 und 1 % liegt.

6. Verfahren nach Anspruch 1, dadurch gekennzeichnet, daß man einen Katalysator vom Typ ZSM-5, vorzugsweise in Form von hydriertem H-ZSM-5, verwendet.

7. Verfahren nach einem der vorangehenden Ansprüche, dadurch gekennzeichnet, daß man mit dem Katalysator eine ethanolische Flüssigkeit, enthaltend mindestens 4 Gew.-% Ethanol, umsetzt.

8. Verfahren nach Anspruch 7, dadurch gekennzeichnet, daß die ethanolische Flüssigkeit eine Flüssigkeit ist, die erhalten worden ist durch Fermentation einer Biomasse oder eines Synthesegases, das erhalten worden ist aus Kohle oder Erdöl, enthaltend Ethanol.